(19) Europäisches Patentamt
European Patent Office
Office européen des brevets

(11) **EP 3 662 054 B1**

(12) **EUROPEAN PATENT SPECIFICATION**

(45) Date of publication and mention of the grant of the patent:
**03.08.2022 Bulletin 2022/31**

(51) International Patent Classification (IPC):
**C12N 5/00** (2006.01)   **C08J 3/075** (2006.01)
**C08J 3/24** (2006.01)

(21) Application number: **18730009.0**

(22) Date of filing: **18.06.2018**

(52) Cooperative Patent Classification (CPC):
**C12N 5/0062; C08J 3/075; C08J 3/24;**
C08J 2300/14; C08J 2333/02; C12N 2533/40;
C12N 2533/78

(86) International application number:
**PCT/EP2018/066096**

(87) International publication number:
**WO 2019/025070 (07.02.2019 Gazette 2019/06)**

(54) **A THREE-DIMENSIONAL HYDROGEL SCAFFOLD FOR CELL CULTURING AND A METHOD FOR THE PRODUCTION THEREOF**

DREIDIMENSIONALES HYDROGELGERÜST FÜR ZELLKULTUR UND VERFAHREN ZU DESSEN HERSTELLUNG

ÉCHAFAUDAGE D'HYDROGEL TRIDIMENSIONNEL POUR CULTURE CELLULAIRE ET SA MÉTHODE DE PRODUCTION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.07.2017 IT 201700087978**

(43) Date of publication of application:
**10.06.2020 Bulletin 2020/24**

(73) Proprietor: **Universita' degli Studi di Genova 16126 Genova (IT)**

(72) Inventors:
• **CAVIGLIOLI, Gabriele**
  **16148 Genova (IT)**
• **BALDASSARI, Sara**
  **16148 Genova (IT)**
• **ZUCCARI, Guendalina**
  **16148 Genova (IT)**
• **BASSI, Anna Maria**
  **16132 Genova (IT)**
• **YAN, Mengying**
  **16148 Genova (IT)**

(74) Representative: **Ferreccio, Rinaldo**
**Botti & Ferrari S.p.A.**
**Via Cappellini, 11**
**20124 Milano (IT)**

(56) References cited:
**WO-A1-2016/168196     WO-A1-2017/033892**
**US-A1- 2007 098 799**

• **NASIM ANNABI ET AL: "Controlling the Porosity and Microarchitecture of Hydrogels for Tissue Engineering", TISSUE ENGINEERING PART B-REVIEWS, vol. 16, no. 4, August 2010 (2010-08), pages 371-383, XP055437447, US ISSN: 1937-3368, DOI: 10.1089/ten.teb.2009.0639 cited in the application**
• **SHENGTONG SUN ET AL: "Hydrogels from Amorphous Calcium Carbonate and Polyacrylic Acid: Bio-Inspired Materials for "Mineral Plastics"", ANGEWANDTE CHEMIE INTERNATIONAL EDITION, vol. 55, no. 39, 22 July 2016 (2016-07-22) , pages 11765-11769, XP055437354, ISSN: 1433-7851, DOI: 10.1002/anie.201602849 cited in the application**

EP 3 662 054 B1

- JUNMIN ZHU: "Bioactive modification of poly(ethylene glycol) hydrogels for tissue engineering", BIOMATERIALS, vol. 31, no. 17, June 2010 (2010-06), pages 4639-4656, XP055112904, ISSN: 0142-9612, DOI: 10.1016/j.biomaterials.2010.02.044 cited in the application
- Fang Ren: "Synthesis of Gold Nanoparticle-Hydrogel Nanocomposites with Controlled Cytotoxicity and Unique Cell-Adhesive Properties", , 9 September 2016 (2016-09-09), pages 1-132, XP055437375, Retrieved from the Internet: URL:https://depositonce.tu-berlin.de/bitst ream/11303/5988/4/ren_fang.pdf [retrieved on 2017-12-21] cited in the application
- ENAS M. AHMED: "Hydrogel: Preparation, characterization, and applications", JOURNAL OF ADVANCED RESEARCH, July 2013 (2013-07), XP055163462, ISSN: 2090-1232, DOI: 10.1016/j.jare.2013.07.006
- MARK W. TIBBITT ET AL: "Hydrogels as extracellular matrix mimics for 3D cell culture", BIOTECHNOLOGY AND BIOENGINEERING, vol. 103, no. 4, July 2009 (2009-07), pages 655-663, XP055314314, ISSN: 0006-3592, DOI: 10.1002/bit.22361

**Description**

Field of application

This invention relates to the fields of material technology for cell culturing and tissue engineering.

In particular, the present invention relates to a method for the production of an unerodible, transparent and sterilizable hydrogel scaffold for a three-dimensional cell culture which can be used to culture a variety of different cells and tissues in vitro for prolonged periods of time.

Prior art

In many chemical technological fields, efficacy and toxicity studies are required for pure substances and formulations. Health surveillance and regulatory agencies in many countries are urging for the development of alternative methods to replace experiments on animals in the aforementioned areas. Thus, development of more reliable cell cultures to mimic the in vivo biological environment, cross-talk among different cells/tissues/ organs need to be developed and performed worldwide.

Cell culture is the process by which cells are grown under controlled conditions, generally outside their natural environment. Nowadays, the term "cell culture" refers to the culturing of cells derived from multi-cellular eukaryotes, especially animal cells, but the culturing can be extended to other growing cells, such as plant tissue culture, fungal and microbiological culture.

Cells can be grown either in suspension or as adherent cultures. Some cells naturally live in suspension, without being attached to a surface, such as blood cellular components. There are also cell lines that have been modified to be able to survive in suspension, so they can be grown to a higher density than adherent conditions would allow. Adherent cells require a surface, such as tissue culture plastics or microcarriers, which may be coated with extracellular matrix components (such as collagen, laminine, polylysine) to improve adhesion properties and provide other signals needed for growth and differentiation. Plating density (number of cells per volume of culture medium) plays a critical role for culturing some cell types: cells generally continue to divide in culture until the available area or volume is filled.

Traditional methods of cell culture have been studied on 2-dimensional (2D) surfaces such as Petri dishes, tissue culture flasks, and micro-well plates. It has been described that the cultured cells respond to environmental stimuli, and when in contact with a plastic support they normally form a monolayer, where the mutual interactions occur only along the cell edges [1, 2]. In 2D models only 50% of the cell surface is in contact with the culture medium. Moreover, the cells tend to flatten on the scaffold surface, with consequent morphological changes occurring relatively early during culture growth, that may induce alterations in cellular biochemical processes and trafficking. In fact, the two-dimensional growth apparently influences the gene and protein expression, with significant differences in cellular responses from those observed in vivo, possibly causing abnormally negative or positive assay outcomes.

A "three-dimensional cell culture" or also named "3D cell culture" allows cells in vitro to grow in all directions, similar to in vivo condition.

Three-dimensional cell cultures have greater stability and longer lifespans than cell cultures in 2D. Also, 3D aggregates can be cultured for longer, also more than two weeks, as compared to almost one week with 2D monolayer culture due to cell confluence. Therefore, they might be more appropriate for long-term studies and surviving cells and for demonstrating long-term effects of the drug. Thanks to the 3D environment, the cells are allowed to grow undisturbed compared to the 2D models, where regular dissociation by animal origin (porcine) trypsin, or more recent animal-free recombinant cell-dissociation enzymes, is necessary in order to provide them with sufficient nutrients for normal cell growth.

Increasingly, 2D culture approaches have shown their limitations for new challenges in cell biology and biochemistry studies, as well as in drug discovery and pharmacological studies [3]. It has been proven that 2D culture cannot reproduce completely cell morphology and biochemical features in the original tissue. In order to overcome the 2D cultures shortcomings, research on structures suitable for cell culture three-dimensional growth has been active for a decade. A significant advantage of 3D cell cultures over the 2D methods is their well-represented geometry and chemistry; moreover, they provide a microenvironment more closely resembling the in vivo situation. Actually, 3D cultures, in which the whole cell surfaces can be in contact with the culture medium and with other cells, can mimic better the cross-talk among cells also from different tissues, to reassembly the complex in vivo interactions. These features come out by comparison with 2D cultures, in terms of cell proliferation and differentiation and response to environmental stimuli.

While 2D cell culture monolayers facilitate examination of intracellular signaling cascades and cell behavior, this approach largely ignores the more complex structural organization present in the normal physiological setting. Cells behave differently in a 3D environment, in part because the extracellular matrix is a key regulator of normal homeostasis and tissue phenotype. Examination of cellular behavior and signaling using this 3D cell culture approach improves the relevance of cell-based assays.

As 3D cell culture allows generating 3D aggregates in a non-turbulent environment, with minimal shear force and continually suspended "freefall" culture, it allows the generation of 3D tissue-like aggregates which display physiologically relevant phenotypes, such as lower proliferation rate, hypoxic regions and vasculature.

The 3D cell culture systems let understand the complex cellular physiological mechanisms, and are therefore widely applied in differentiation studies, pharmaceutical assays, tumor models and cancer biology research [4], gene and protein expression studies, besides being applied in tissue engineering, in bioreactor and in vitro modeling of human organs.

Lastly, through analysis of gene expression, microRNA and metabolic profiles, it has been shown that 3D generating aggregates display a genotype significantly more relevant to in vivo, as compared to 2D monolayers cultures [5] .

In the 3D cell culture systems, the cells can grow as spheroids, or 3D cell colonies. 3D spheroids more closely resemble in vivo tissue in terms of cellular communication and the development of extracellular matrixes. These matrixes help the cells move within their spheroid similarly to the way cells would move in living tissue. The spheroids are thus improved models for cell migration, differentiation, survival, and growth and gene/ protein expression[6].

By culturing stem cells or progenitor cells in a 3D culture, it is possible to assist to organoid formation.

An organoid is a miniaturized and simplified version of an organ produced in vitro in three dimensions that shows realistic micro-anatomy. They are derived from one or a few cells from a tissue, embryonic stem cells or induced pluripotent stem cells, which can self-organize in three-dimensional culture owing to their self-renewal and differentiation capacities. Organoids have also been created using adult stem cells extracted from the target organ, and cultured in 3D media [7, 8].

Hydrogels can be used to influence mesenchymal stem cell differentiation towards hard (bone) or soft tissue (neural-like, adipose) cell phenotypes in the absence of differentiation media, immobilized matrix proteins or bioactive motifs. The 3D nature of the gels offers a major opportunity to develop more realistic in vitro models for cancer research. Transwell or collagen overlay assays are standard methods used to measure macrophage and tumor cell co-migration. Hydrogels can be suitable to create a reliable, more in vivo-like culture system that opens up to new possibilities in cancer research, from mimicking ECM environment to designing multi-cellular, tumor-like systems, to developing culture systems that favor tumor stem cells. In these microenvironments also monocyte-derived cells, as macrophages, can be co-cultured with tumor cells into a 3D structure, to mimic the pathophysiological development of tumors in vivo, and tumor cell-macrophage interactions in the promotion of tumor invasion.

Cell cultures on hydrogels are already finding applications in the following areas: cell-based assays, toxicity screening, cancer cell research, biofabrication, micro/millifluidics, regenerative medicine.

There are a number of commercially available culturing tools for 3D cell culture, which can be classified in two types: scaffold techniques and scaffold-free techniques.

Scaffold-free techniques employ another approach independent from the use of scaffolds.

The scaffolds currently used may be classified in solid structures and hydrogel matrixes.

The main requirements for 3D cell culture scaffolds are: high compatibility with most cell lines, suitable mechanical strength, optical transparency, high porosity and interconnectivity, sterilizability, manageability, hydrophilicity, easy diffusion of substances to be tested and gas, easy dissection into thin layers, absence of interference with conventional assays for cell growth evaluation, easy production and low cost [9-12]. However, the optimal 3D scaffold combining the most appropriate characteristics has not been developed yet.

Solid scaffolds are normally made of either inorganic (calcium phosphate) or organic polymeric materials (polystyrene). Their main shortcomings are: they are not transparent (therefore limiting cell observation during culture growth), difficult to sterilize, not suitable to hold cells; moreover, cell detachment and recovery to perform viability tests or to move the culture to another support are labor intensive and allow only a partial recovery of the cells. This type of scaffolds may be applied in immunohistochemistry, electronic microscopy, fluorescence microscopy, confocal microscopy, cytofluorimetry, biochemical assays, protein and nucleic acid production and extraction, analysis of molecules synthesized by various cell lines.

Hydrogels are three-dimensional, cross-linked polymeric networks which can swell in water forming soft elastic materials, and retain a significant fraction of water in their structure without dissolving.

They can be employed for tissue engineering, since their structures are soft, easily hydrated and several hydrogels can be tuned to resemble specific tissues. These features support very well the transport/exchange of nutrients, and water-soluble factors, as removal of catabolites. With respect to polymeric scaffolds, the hydrogels are commonly quite transparent allowing to a sufficient extent the possibility of microscopy observation, during culture time, to check the healthy state of cells. However, hydrogels have to be maintained in a hydrated state, therefore they could possibly suffer from long term stability issues in vitro and some densely crosslinked gels can hinder cellular migration.

The scaffolds based on polymeric hydrogels are more similar to the extracellular matrix (ECM) of living tissues and porous scaffolds based on hydrophilic polymer networks can mimic in vivo tissue architecture, therefore being more favorable for organotypic cell proliferation [13]. Yet, their weaknesses can be: inadequate mechanic strength or weak rheological consistency, difficult cell attachment, complex and expensive preparation costs.

Porous materials are classified into several kinds by their size. According to IUPAC notation [14], microporous materials have pore diameters of less than 2 nm and macroporous materials have pore diameters of greater than 50 nm; the mesoporous category thus lies in the middle.

Good mechanical properties allow easier handling of the scaffold during the process of cell culture; moreover, the flexibility of the scaffolds has demonstrated to play a crucial role in increasing gene expression for the cultured cells [14].

Hydrogels are the most promising option for cell culture and have proven useful in a range of cell culture applications, revealing fundamental phenomena regulating cell behavior and providing tools for the expansion and directed differentiation of various cell types in ways not possible with conventional 2D cultures. For example, in 3D hydrogel mammary epithelial cell assemble into multicellular spherical structures resembling healthy acini; pluripotency markers of embryonic stem cells can be controlled over mechanic properties or chemical modifications; in peptide hydrogel cells show improved resistance to chemotherapeutics, neurons with genetic mutations related to Alzheimer's disease show the formation of the lesions typical of this pathology, as the amyloid plaques and the neurofibrillary tangles [15], [16].

Three-dimensional scaffolds can be prepared by different techniques, such as gas-forming foam, three-dimensional printing, thermally-induced phase separation, electrospinning, freeze-drying, solvent casting, compression moulding, immersion precipitation, particulate leaching, supercritical $CO_2$ technique, free-radical polymerization or copolymerization, double network polymerization, templating fabrication.

Many hydrogels can encapsulate seeded cells after their fabrication or the viable cells can be encapsulated with the hydrogel precursor that generates the hydrogels by chain-growth or step-growth polymerization. It is important to use nontoxic initiators (it has been reported that free radicals in the photo-polymerization process can damage the cells) and cross linkers for a rapid gel formation to prevent cell settling during the encapsulation process [15].

Moreover, in all processes using cross-linking agents the cell toxicity of the residual should be always evaluated.

Conventional emulsion templating techniques require large amounts of organic solvent to generate the internal phase (more than 75%), which may be difficult to remove after curing, resulting in cell cytotoxicity.

The porogenic template is a diverse and versatile route for the preparation of porous materials that has been applied over a wide range of length scales. Water is cheap and a number of porous scaffolds for tissue engineering and biological applications have been prepared by freeze-drying, in which ice was used as a porogenic template [17].

However, freeze-drying techniques encounter difficulty in precisely tuning pore size since the hydrogel architectures formed using this method are extremely sensitive to the kinetics of the thermal quenching process; other drawbacks are the low structural stability and generally weak mechanical properties of the surface, because the matrix may collapse at the scaffold-air interface due to the interfacial tension caused by solvent elimination [18].

Other processes to obtain synthetic polymer scaffolds use organic solvents that might remain as residues in the scaffold and being harmful for cultured cells.

From a manufacturing point of view, the preparation of porous structures from the polymer melt or sintering of compacted powders are more convenient routes, as they allow the rapid and economic production of scaffolds of many shapes and sizes [19].

Scaffolds should have high porosity and interconnected porous structure to provide adequate space for the cell seeding, growth and proliferation, in fact, an appropriate micro-architecture of the scaffolds can influence directly the behavior of the cultured cells.

Previous studies reported that scaffolds with porosity higher than 80%, are ideal for uses in 3D cell culture. Some scaffolds show porosity up to 99% as the scaffold containing 1% gelatin described by Wu et al. [17] [20].

However, most existing hydrogel scaffolds with highly interconnected porosity suffer from poor mechanical properties limiting their handling.

As a matter of fact, the compression modulus of the porous structures was found to decrease with an increase in porosity according to power-law relationships. [21]

Most scaffolds can be weak at high porosities (more than 90%) and they can collapse during preparation and in this case it is mandatory to find ways to strengthen them, for example through reinforcing fibers or solid supports. This can be detrimental to other useful properties like transparency, cytocompatibility or sterilizability.

Conventional thermal processing techniques such as sintering and compression moulding of polymer and salt particle mixtures followed by salt leaching normally generate scaffolds with relatively low porosities and at porosities higher than 90% the structures are fragile [21].

Furthermore, it is relatively infrequent that precision control is achieved over pore size and structure of the scaffold, because there are relatively few current technologies, as salt leaching, that allow such control [22].

The capacity of the scaffolds to retain water is another essential parameter of the polymeric network for the permeation of nutrients and waste from the cells. In hydrogels three different types of water can exist, depending on the interactions: free water, water linked through hydrogen bonds and water more strongly linked through polar interactions as with carboxylate group [23]. Water within the hydrogel is mainly stored in pores and channels of the scaffolds and only in part is absorbed in the hydrogel wall. Generally, the elasticity of the hydrogel correlates with the swelling behavior of the hydrogels increasing with the amount of water absorbed.

Natural biomaterials suitable for 3D cell culture in vitro include collagen (Purecol, Fibricol, Flexcell or collagen-containing mixtures such as Matrigel®), alginate, agarose, and fibrin. However, these hydrogels are known to exhibit fast release of loaded growth factors and degrade very quickly, often not allowing adequate time for cell attachment and growth. Synthetic materials, although non-toxic, could be non-suitable for culturing cells: in fact, neuronal cell are known to die in PEG-like hydrogels within 1 day of culture and do not exhibit greater viability or proliferation as compared to monolayer controls [24]; polyacrylamide is suitable for 2D cell culture only [15].

Hybrid materials like hyaluronic acid can influence the cellular experiments because of their numerous cellular interactions, while polypeptides have prohibitive cost for large-scale culture systems and often they are not stable for a long period.

Poly(acrylic acid) (PAA), a weak anionic polyelectrolyte, is considered a pH- and electrically-sensitive material. Hydrogel networks formed from PAA have the ability to absorb many times their weight in water and are the basis of a class of materials called super-absorbents. These polymers are used in many applications, including filters, ion exchange resins, and membranes for hemodialysis, ultrafiltration, and controlled release devices.

Poly(acrylic acid) has a carboxylic acid functionality (or carboxyl group or -COOH), providing a useful substrate for subsequent surface modifications and bio-conjugations [25] to ameliorate the control of cultured cell behaviors: for example, collagen has been immobilized on PAA. which remarkably improved the attachment and proliferation of human dermal fibroblasts and myoblasts [26].

Non cross-linked acrylic polymers [27] have also been disclosed as non-living material enveloped by stromal cells and connective tissue proteins to form a three-dimensional structure having interstitial spaces bridged by the stromal cells.

A patent [28] discloses a polycarbophil conjugate with EGF (Epidermal growth factor) or bFGF (Basic fibroblast growth factor) as attachment and growth promoting agents for growing corneal endothelial cells, for artificial corneal transplants, but not as scaffold and not as thermally-treated polymer.

Sheetz [29] discloses a non-porous scaffold for cell growth comprising a portion of substrate and fiber stretched over the substrate portion, wherein the substrate comprises a growth medium and a cross-linked polymer. Among the useful polymers, polyacrylic acid is only mentioned; but the procedure does not involve thermal treatment or leaching steps in the presence of porigen substances.

Liu et al. [30] disclose a three dimensional solid construct which can be coated with a polyacrylic acid.

Shin et al. [31] disclose a complex support for regenerating bone-cartilage, where a polyacrylic acid can be present in the bone regeneration layer.

Zussman et al. [32] disclose a fiber-reinforced hydrogel containing acrylic polymers usable as scaffolds for cell culture.

Scaffolds used as cell culture or bioreactors must be sterilized to exclude or reduce bacterial contamination, and this aspect represents a critical issue for most part of the scaffold.

Rarely polymer scaffolds can be sterilized by thermal processes or ionizing radiation (gamma and beta-rays) which can be cause of damage to the polymeric skeleton of the scaffolds, or by a chemical process which can contaminate the cells with toxic residues or react with the polymers used in the scaffold. Furthermore, the largely employed ultraviolet irradiation is only an aid in the reduction of contamination only on the exposed surface because of its negligible penetration. Chemical disinfection (ethylene oxide, ethanol) can alter scaffold properties and leave toxic residues. Polymeric hydrogel can rarely resist to terminal sterilization by classical thermal methods, such as moist heat sterilization. For this reason the large majority of 3D scaffolds must be produced by expensive aseptic procedures [15].

Sponges are porous structures whose properties are determined by interconnectivity, pore size, and porosity, which impact cell penetration and migration, matrix deposition and distribution, and nutrient and waste exchange. A hydrogel is a network of polymer chains that are hydrophilic, swollen polymer matrixes with a cross-linked structure and which retain a large amount of water. Sponges are defined by morphology, while hydrogels are defined by hydrophobicity. According to some authors, hydrogels are made of hydrophilic materials with water contact angles less than 60°, while sponges are porous structures made of materials with a water contact angle of 80° or above. Macroporous hydrogel sponges can be used as scaffolds for 3D culturing cells, tissue engineering and in the bioreactor technology [33],[30]. Also filling hydrogels into sponges have been reported to be a very efficient tool for 3D culture in scaffold [34].

Bogdanowicz et al., [35] describe a model which allows for the study of the effects of both cell-cell contact and paracrine signaling on subpopulations of cells in co-culture; moreover, the model exerts spatial and temporal control over heterotypic cellular interactions through the development of a removable permeable hydrogel barrier, permitting the live tracking of populations throughout in vitro culture and allowing for the study of individual cell subpopulation response to co-culture.

Caliari et al [15] describe the use of hydrogels to study cell viability, morphogenesis, aging, and disease, with a focus on commercially available hydrogels.

Nasim Annabi et al. "Controlling the porosity and Microarchitecture of Hydrogels for Tissue Engineering", Tissue Engineering part B-Reviews, vol. 16, no. 4, August 2010, pages 371-383, disclose hydrogels for tissue engineering. In particular, by using sodium bicarbonate to fabricate an acrylic acid-acryl amide porous hydrogel by crosslinking acrylic acid and acrylic amide with N-N'-methylenebisacrylamide, a porous hydrogel having an interconnected structure with pore size ranging from 100 to 250 $\mu$m was obtained.

Shengton Sun et al., "Hydrogels from amorphous calcium carbonate and polyacrylic acid: bio-inspired materials for "mineral plastics", Angewandte Chemie International Edition, vol. 55, no. 39, 22 July 2016, pages 11765-11769, disclose an amorphous $CaCO_3$-based hydrogel consisting of very small particles of amorphous calcium carbonate physically cross-linked by poly(acrylic acid).

Junmin Zhu, "Bioactive modification of poly(ethylene glycol) hydrogels for tissue engineering", Biomaterials, vol. 31, no. 17, June 2010, pages 4639-4656, is a review concerning the progress in material design and fabrication approaches leading to the development of bioactive hydrogels as tissue engineering scaffolds Fang Ren, "Synthesis of gold nanoparticle-hydrogel nanocomposites with controlled cytotoxicity and unique cell-adhesive properties", 9 September 2016, pages 1-132,https://depositonce.tu-berlin.de/bitstream/11303/5988/4/ren_fang.pdf, discloses the immobilization of gold nanoparticles on cyto-compatible hydrogels to synthesize novel nanocomposite hydrogels, which have been applied in cancer treatment and to control cell behavior on cell adhesion.

WO 2016/168196 A1 discloses water-insoluble but water-swellable and deformable crosslinked PEGylated microgel particles of proteins and protein-based macromolecules tha are pseudoplastic and flow in aqueous medium under shear. These microgel particles function as a matrix to support cell growth, viability and proliferation.

US 2007/0098799 discloses mineralized hydrogels obtained by combining a first mixture including a calcium derivative, a second mixture including a phosphate derivative and a hydrogel to form a calcium phosphate dispersion containing the hydrogel. The hydrogel in the calcium phosphate dispersion is crosslinked to form a mineralized hydrogel, in which calcium phosphate minerals are substantially dispersed within the hydrogel.

The objective technical problem underlying the present invention is to provide a method for the production of a three-dimensional scaffold suitable for culturing cells for a long time, which, while being unerodable in aqueous mediums, has a high optical transparency allowing a microscopic observation of cell growth and morphology, and also has high molecular diffusion to permit the use in situ of agent for cell viability evaluation test or for standard immunostaining protocols and suitable mechanical properties to be easily handled. It should also be resistant to the energy sterilization process or to chemical agents.

## SUMMARY OF THE INVENTION

This problem has been solved, according to the invention, by the method for the preparation of a three-dimensional hydrogel scaffold for cell culturing as disclosed in appended claim 1.

Preferred embodiments of this method are disclosed in dependent claims 2-15

The thermal treatment carried out on the compact units obtained from the above-mentioned solid mixture generates a robust 3D matrix which can be submitted to the leaching treatment with deionized water to become highly porous.

Compact units produced according to steps a) and b), but not subjected to the thermal treatment of step c) cannot undergo the leaching treatment of step d), because they disintegrate when contacted with water.

In order to control porosity, pore shape and size, the at least one water-soluble salt and the at least one gas-forming agent can be used as powder with a size controlled by milling and sieving procedures.

The present method does not use any organic solvent or cross-linking agent, and produces a scaffold with the above-mentioned properties suitable for culturing different kinds of the cells even for a long time (longer than 2D), allowing a good permeation of gases, nutrients and waste from the cells.

The three-dimensional scaffold obtained with the method according to the present invention involves several advantages.

The gas-forming agent improves the structure and increases the interconnectivity and porosity of the three-dimensional scaffold, thus guaranteeing and promoting cell and small molecules movement through the three-dimensional structure.

The water-soluble salt improves micro-porosity and interconnectivity of the above-mentioned scaffold.

Advantageously, the scaffold obtained with the method of the invention can be used in conventional cell culture vessels, such as cell culture dishes, cell culture plates, cell culture flasks, micro-well plates, cell culture bags, bioreactors and microfluidic devices.

Due to its high molecular diffusion, said scaffold permits the use in situ of agents for cell viability evaluation test or for standard immune-staining protocols.

Therefore, the scaffold with cultured cells may be employed in a variety of applications ranging from cytotoxical and pharmacological screening, assaying the biological activity of compounds in vitro, studying the growth, proliferation, and function of cells, and the production of biologically active molecules, as biological model of tissues, for growing tissue and for in vivo implantation o transplantation of tissue (bone, bone marrow, liver, mucosal epithelium, pancreas, brain, lung, vascular, hearth, gut, intestinal, membranous, retina, eyes, cartilaginous etc).

The scaffold obtained with the method of the present invention may be used to grow other kinds of cells, such as plant tissue culture, mycological and microbiological culture (of microbes). For example, the 3D scaffold according to the invention can be used for culturing and studying microbiota that normally inhabits the skin, mouth, nose, digestive tract, and vagina.

Moreover, the scaffold obtained with the method of the present invention resists to the high temperature and pressure of the sterilization treatment by moist-heat autoclave, maintaining the mechanical and morphological properties and the suitability for culturing cells.

Brief description of the drawings

Figure 1A shows a schematic view of a three layers compacted unit.

Figure 1B shows a schematic view of a partially dry-coated unit, with only lower and lateral coating without upper coating layer.

Figure 2 shows C2 before (left) and after (right) thermal treatment.

Figure 3 shows C2 surface after thermal treatment.

Figure 4 shows a comparison of a C2 scaffold (on the left) and a C2 unit that has been not submitted to thermal treatment (on the right) after leaching with water, showing an evident disaggregation caused by the leaching procedure.

Figure 5A shows SEM micrograph of cross section of thermally-untreated C2 compacted unit.

Figure 5B shows SEM micrograph of cross section of thermally-treated C2 compacted unit.

Figures 6A, 6B and 6C show SEM micrographs of cross sections of thermally treated C2 compacted unit.

Figure 7A shows SEM micrograph of cross section of thermally-untreated C5 compacted unit.

Figure 7B shows SEM micrograph of cross section of thermally-treated C5 compacted unit.

Figures 8A and 8B show SEM micrographs of cross sections of thermally treated C5 compacted unit.

Figures 9A, 9B and 9C show SEM micrographs of cross sections of lyophilized C2 matrix after leaching, conditioning and sterilization in PBS.

Figures 10A and 10B show SEM micrographs of cross sections of lyophilized C2 matrix after leaching and sterilization in water.

Figure 11 shows SEM micrograph of cross section of lyophilized C5 matrix after leaching, conditioning and sterilization in PBS.

Figure 12 shows SEM micrograph of cross section of lyophilized C5 matrix after leaching and sterilization in water.

Figure 13 shows Quartz UV cuvette filled with cut cubes of C2 PBS-conditioned scaffold (left) and pure Carbopol 980 PBS-swollen matrix (right).

Figure 14 represents a graph showing 300-800 nm spectra for pure Carbopol 980 (Cbp980), Carbopol 974 (Cbp974p), polycarbophyl (pol) thermally treated compacted units, and C2, C8 hydrogel sponge scaffolds, conditioned in PBS and sterilized.

Figure 15 shows transparency of C2 (centre) and C8 (right) hydrogel sponge scaffolds with respect to PBS solution (left).

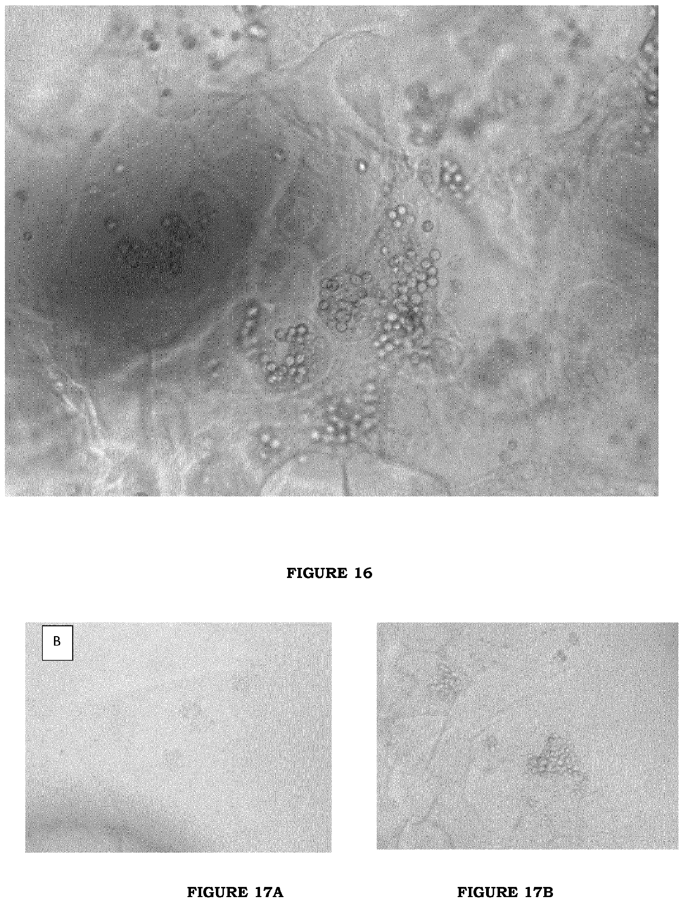

Figure 16 shows HaCat keratinocytes organized in a cluster structure, being arranged inside the space of hydrogel sponge scaffold C2 showing the tendency to form compact spheroids, after 72 hours of seeding into C2 scaffold. (Contrast phase microscopy, magnification 10X).

Figure 17A shows HECV cells after 72 hours in K8 resulting in a morphology similar to spheroids reassembling a tube. (Contrast phase microscopy, magnification 10X).

Figure 17B shows HECV cells after 72 hours in C8 resulting in a morphology similar to spheroids reassembling a tube. (Contrast phase microscopy, magnification 10X).

Figure 18 shows epithelial HeLa cells which appear dispersed in hydrogel sponge scaffold P8, not forming a compact cluster probably for their undifferentiated phenotype (Contrast phase microscopy, magnification 10X).

Figure 19 shows a graph representing stress-strain curve of scaffolds.

Figure 20 shows a graph representing stress-strain curve of scaffold C2.

Figure 21 shows a graph representing stress-strain curve of scaffold C8.

Figure 22 shows a graph representing an isothermal plot for scaffold C2.

Figure 23 shows a graph representing BET surface plot of scaffold C2.

Figure 24 shows a graph representing BJH dV/dD pore volume of scaffold C2.

Figure 25 shows a graph representing IR spectrum of P11 in KBr

Figure 26 shows a graph representing IR spectrum of K2 in KBr.

Figure 27 shows a graph representing IR spectrum of C12 in KBr.

Figure 28 shows the gels just placed into Petri dishes: on top C2 gels, on bottom C8 gels.

Figure 29A A shows the phenol red spread into the gels after 4 hours of incubation (top panels: C2 gels, bottom panels: C8 gels): within 4 h exposure phenol red resulted into all the gels.

Figure 29B shows the phenol red spread into the gels after 24 hours of incubation (top panels: C2 gels, bottom panels: C8 gels): within 24 h exposure phenol red resulted into all the gels.

Figure 30A shows C2 and C8 gels, without cells, just placed into 6 multi-wells and exposed to MTT, MTS and NRU viability dyes.

Figure 30B shows C2 and C8 gels, without cells, after 3 hour-exposure to MTT, MTS and NRU viability dyes.

Figure 31A shows a bar graph representing 20 days long viability of HeLa cells cultured in C2 and C8 hydrogel sponge scaffolds and tested by Alamar Blue®. Data are the mean of three separate experiments ± SD, run in duplicate.

Figure 31B shows a bar graph representing 20 days long viability of HECV cells cultured in C2 and C8 hydrogel sponge scaffolds and tested by Alamar Blue®. Data are the mean of three separate experiments ± SD, run in duplicate.

Figure 32A shows a bar graph representing MTS response to nickel sulfate irritation test for HeLa cell line cultured in C8 scaffold after ten days; the viability indexes are expressed as mean of two genuine performed in duplicate ± SD.

Figure 32B shows a bar graph representing MTS response to nickel sulfate irritation test for HeLa cell line cultured in C2 scaffold after ten days; the viability indexes are expressed as mean of two genuine performed in duplicate ± SD.

Figures 33A and 33B show sections cut from C2 hydrogel sponge scaffold, embedded in agarose with PI-stained HECV cell line cultured-in for 20 days at fluorescence microscope; cells have prolongations (arrows).

Figure 34 shows sections cut from C8 hydrogel sponge scaffold, embedded in agarose with DAPI-stained HECV cell line cultured-in for 20 days at fluorescence microscope.

Figure 35A shows sections close to superior surface of the scaffold (ScS) which were cut from C8 hydrogel sponge scaffold, embedded in agarose with HeLa cell line cultured-in for 20 days; cells are visible in the whole thickness (arrows).

Figure 35B shows sections close to the bottom of the scaffold which were cut from C8 hydrogel sponge scaffold, embedded in agarose with HeLa cell line cultured-in for 20 days at light microscope. Cells are visible in the whole thickness (arrows).

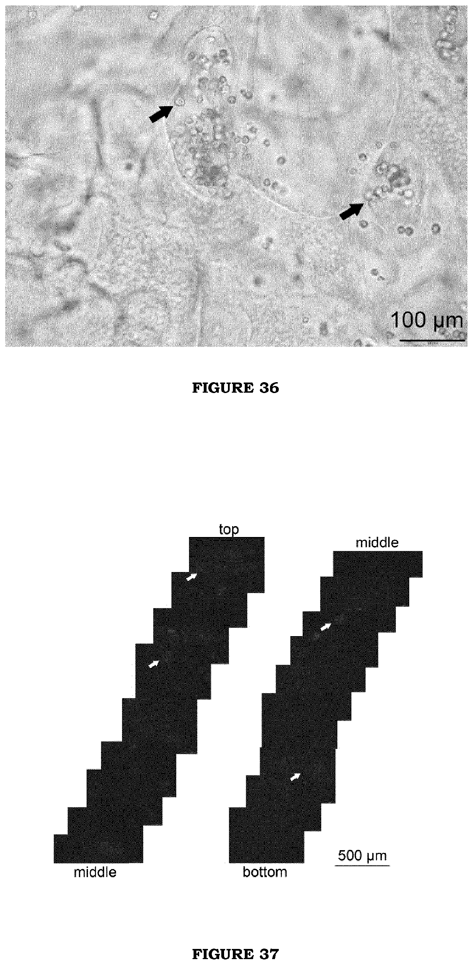

Figure 36 shows a section cut from C8 hydrogel sponge scaffold, embedded in agarose with HeLa cell line cultured-in for 20 days at light microscope. Cells (arrows) are clustered.

Figure 37 shows sections cut from C8 hydrogel sponge scaffold, embedded in agarose with IP-stained HeLa cell line cultured-in for 20 days at fluorescence microscope; the sequence of the sections ranging from the seeding surface to the bottom of the scaffold is reported. Cells (arrows) are distributed.

Figure 38 shows a section cut from C8 hydrogel sponge scaffold, embedded in agarose with IP-stained HeLa cell line cultured-in for 20 days at fluorescence microscope. Cells (arrows) are clustered.

DETAILED DESCRIPTION OF THE INVENTION

The present invention relates to a method for the preparation of a three-dimensional hydrogel scaffold for cell culturing, which comprises the steps of:

a) preparing at least one first uniform solid mixture comprising at least one gas-forming agent, selected from the group consisting of sodium hydrogen carbonate, potassium hydrogen carbonate, sodium glycine carbonate, ammonium hydrogen carbonate, sodium bisulfite, sodium metabisulfite, calcium carbonate and sodium sulfite, at least one water-soluble salt and at least one non-water-soluble, water-swellable cross-linked poly(acrylic) polymer;

b) preparing compressed or compact units starting from said at least one first solid mixture;

c) subjecting the compressed or compact units thus obtained to heating at a temperature in the range of 120°C-160°C, for a time of 1-120 minutes, preferably 1-20 minutes;

d) leaching the thermally treated units thus obtained in an aqueous medium;

e) conditioning the leached units thus obtained in water or in an aqueous solution selected from the group comprising a buffer solution, preferably phosphate-buffered saline (PBS) and a cell culture medium;

f) subjecting the conditioned units thus obtained to sterilization.

The scaffold obtained by this method can be used to culture different cellular types in mixed co-culture models or segregated co-culture systems.
In particular, the 3D scaffolds obtained by the method of the present invention are useful for culturing cells derived from multi-cellular eukaryotes, especially animal cells or genetically-modified cells or other types of cell culture such as plant tissue culture, fungal culture, and microbiological culture.
When conditioned in an aqueous medium, the scaffold appears to be a highly transparent sponge, through which it is possible to observe and study the cell cultures growing in the pores of the structure.
The expressions "initial composition" or "first uniform solid mixture" as used herein mean a mixture comprising a polymeric component, namely a cross-linked poly(acrylic) acid powder, which is hydrophilic but not soluble in water, and a fraction including a thermostable water-soluble salt and a gas-forming agent.

In particular, said cross-linked poly(acrylic) acid may be a polymer of acrylic acid cross-linked with either allyl sucrose or allyl ethers of pentaerythritol like Carbomers (as defined by British Pharmacopeia (BP), European pharmacopoeia Ph. Eur., or US Pharmacopeia (USP) or Handbook of Pharmaceutical Excipients sixth edition, 2009 (UK ISBN 978 0 85369 792 3) and their salts, which is identified by CAS registry number [9003-01-4]. Alternative CAS registry numbers have been used for Carbomer 934 ([9007-16-3]), 940 ([9007-17-4]) and 941 ([9062-04-08]). The CAS registry number [9007-20-9] has also been used for Carbomer.

The cross-linked poly(acrylic) acids may also be polymers of acrylic acid cross-linked with divinyl glycol like Polycarbophil [CAS number 9003-97-8] or Noveon AA-1 and its salts.

The cross-linked polymers of acrylic acid can be used in an amount between 20-80% (w/w) of the initial mixture used to produce the compacted unit. Preferably, the cross-linked polymers of acrylic acid are used in an amount between or 22-60% or 30-50% (w/w) of the initial mixture.

The above-mentioned thermostable water-soluble salt is preferably selected from the group comprising: sodium chloride (NaCl), potassium chloride (KCl), potassium sulfate ($K_2SO_4$), sodium sulfate ($Na_2SO_4$), potassium dihydrogen phosphate ($KH_2PO_4$), disodium hydrogen phosphate ($Na_2HPO_4$), magnesium sulfate ($MgSO_4$), aluminum chloride ($AlCl_3$), magnesium chloride ($MgCl_2$), potassium acetate ($KC_2H_3O_2$) or a mixture thereof. More preferably, the thermostable water-soluble salt is sodium chloride or potassium chloride.

According to an aspect of the invention, said salts may be used in combination with non-ionic water soluble molecules (such as, for example, carbohydrates or polymers), provided that they are stable to the subsequent thermal treatment.

Sodium chloride or any other thermostable water-soluble salt can be used in an amount between 0.5-79.5 % (w/w) of the initial composition preferably 10-50% (w/w), even more preferably 20-40% (w/w).

According to an aspect of the invention, the gas forming agent may be thermolabile.

The above-mentioned thermolabile gas-forming agent is able to generate gas upon the heating of said thermal treatment (up to temperature of about or less than 160°C) according to the method of the present invention. The thermolabile gas-forming agent may be sodium hydrogen carbonate or another thermolabile compound which is able to generate gas, such as, for example, potassium hydrogen carbonate, sodium glycine carbonate, ammonium hydrogen carbonate, sodium bisulfite or sodium metabisulfite.

The gas-forming agent improves the structure and increases the porosity and interconnectivity, such as sodium hydrogen carbonate or any thermolabile or thermostable gas-forming agent.

In another aspect, the gas-forming agent may be a thermostable compound, such as calcium carbonate or sulfites, in particular sodium sulfite. The decomposition of such thermostable gas-forming agent occurs in an acidic solution, because it does not decompose during the thermal treatment of the method according to the present invention (as instead the above-mentioned thermolabile gas-forming agent does). Therefore, in this particular embodiment, the step of gas forming occurs after the thermal treatment step and during the leaching step in a suitable acidic aqueous medium before the conditioning step (pH 0.1-3).

Sodium hydrogen carbonate or other gas-forming agent could be used in a percentage between 0.5-79.5% (w/w) of the initial composition, preferably 10-50% (w/w), even more preferably 20-40% (w/w).

**Three-dimensional scaffold properties**

The 3D scaffolds obtained with the method of the present invention have a porosity higher than 85%, preferably higher than 95% and a pore size comprised between 0.1 nm and 600 $\mu$m, as shown in the Examples.

The Young's modulus, also known as the elastic modulus, of 3D hydrogel sponge scaffolds conditioned in PBS ranges from 5 to 200 kPa (15-120 or 20-110), while yield strength/weight of PBS-conditioned scaffolds ranges from 1 to 30 kPa/g or from 2 to 18 or 3 to 13 (see example 6).

The apparent density of the lyophilized 3D scaffold obtained by freeze-drying from the hydrogel sponge conditioned in water ranges from (see example 4) 5 mg/ $cm^3$ to 50 mg/$cm^3$, preferably 10 mg/ $cm^3$ to 45 mg/$cm^3$, more preferably 12 mg/ $cm^3$ to 40 mg/ $cm^3$, even more preferably 13 to 30 mg/ $cm^3$, being the most preferred the range between 14 mg/ $cm^3$ and 25 mg/ $cm^3$.

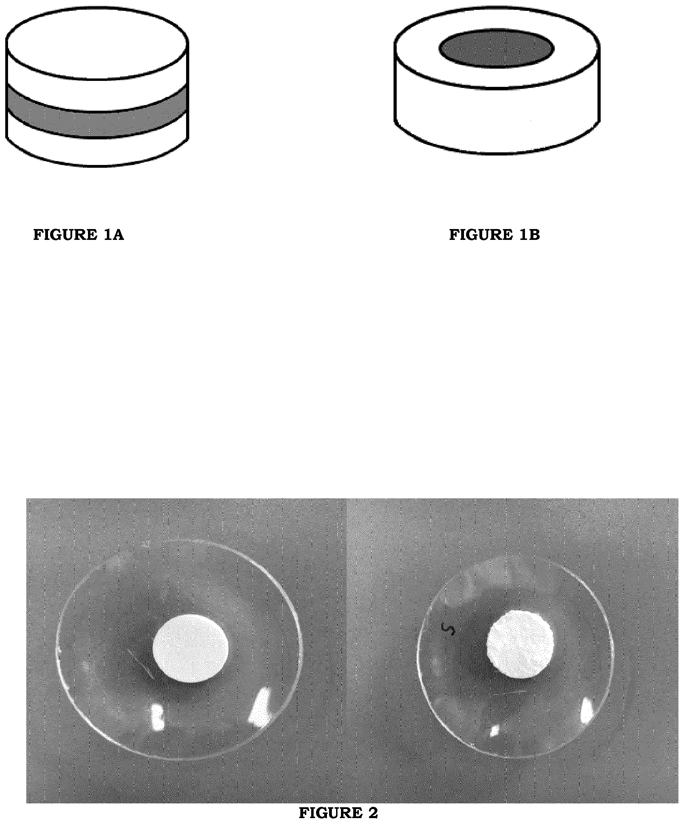

According to an aspect of the method of the present invention, the compacted units can be multilayer units produced by step by step compaction of different layers with different composition, each containing at least one cross-linked polyacrylic acid. The layers can have different or equal composition and contain the same or different cross-linked polyacrylic acid. Some layers, for example the external top or bottom layers, can contain only a cross-linked polyacrylic acid as barrier layers. The compacted multilayer units are submitted to the same preparative steps of thermal treatment, leaching, medium/solvent conditioning, optional vacuum chamber conditioning, optional lyophilization, and sterilization. The different layers can be used to culture different cellular types in mixed co-culture models or segregated co-culture systems [35] (Figure 1A).

According to an aspect of the method of the present invention, the compacted units can also be partially dry-coated, so that the compacted units are made only of lower and lateral coating without upper coating layer. The coating layers are applied by pressure in a tableting machine by centering a pre-compacted cylindrical core in a cylindrical die containing the powder bed of the coating mixture. The coating layers can have different or equal composition and contain the same or a different cross-linked polyacrylic acid. Both the coating layer and the core of the coated unit must contain at least a cross-linked polyacrylic acid. The coating layers can contain only a cross-linked polyacrilic acid as barrier layer. The partially coated units are submitted to the same preparative steps of thermal treatment, water leaching, medium/solvent conditioning, optional steps of vacuum chamber conditioning or lyophilization and sterilization (Figure 1B).

The lyophilized scaffold obtained with the method of the present invention can also be partially film-coated by film forming agents like hydroxypropylcellulose (HPC), ethylcellulose (EC), hydroxyethylmethylcellulose (HEMC), hydroxypropylmethylcellulose (HPMC), methylcellulose (MC), polyvinylalcohol (PVA), polyvinylalcohol acetate phthalate, polymethacrylates (i.e. Eudragit)

The at least one water-soluble salt (for example sodium chloride) and the at least one gas-forming agent (for example, sodium hydrogen carbonate), can be granulated together by one of the procedures well-known in the art, to obtain a porogenic template, generating pores with controlled or homogenous size by thermal treatment and subsequent leaching.

For example, by means of a fluid-bed process it is possible to spray an aqueous sodium chloride solution against a fluid bed of sodium hydrogen carbonate powder to obtain a macroaggregate with a controlled size, for example through sieving, between 50 to 500 $\mu$m. This macroaggregate, after assaying its composition, can be used as template in the scaffold preparation to obtain a more regular porosity in the resulting hydrophilic sponge of 3D scaffold. The sodium chloride solution can contain up to 25% (w/w) of ethanol.

By spray drying an aqueous solution containing the two above-mentioned components, namely the at least one water-soluble salt and the at least one gas-forming agent, at the same concentration (from 5 to 15 %) it is possible to obtain a mixed macro-aggregated powder with controlled size, usable as scaffold template. The aqueous solution of the two components for spray drying can contain up to 25% (w/w) of ethanol.

By granulation and spheronization process followed by a sieving step, it is possible to obtain microspheres of a mixture of the at least one water-soluble salt and the at least one gas-forming agent with controlled sizes to use as porogenic template tableting with the other component (cross-linked polyacrylic acid).

With the scaffold obtained by the method of the present invention, all culturing media or growing media known in the art may be used depending on the type of cells to be grown (including, but not limited to, HBSS = Hank's balanced salt solution; MEM: minimal essential medium; DMEM: Dulbecco's Modified Eagle Medium; RPMI: Roswell Park Memorial Institute medium) and they can contain all those factors used in the cell culture like fetal calf serum, L-glutamine, antibiotics (e.g. Pen-Strep, gentamycine), antifungals (e.g. amphotericin B) and supplementary growth factors.

According to an aspect which is not part of the present invention, the hydrogel sponge or 3D scaffold can be filled with solution or gel (also obtainable in situ) based on artificial and natural biomaterials as hyaluronic acid, collagen (all types), Matrigel® matrix (a gelatinous protein mixture secreted by Engelbreth-Holm-Swarm mouse sarcoma cells and marketed by Corning Life Sciences and BD Biosciences), alginate, gelatin, agarose, chitosan, chitosan-glycerophosphate, bioactive peptides, polyethylene glycol (PEG) modified with either hydrolysable units or bioactive peptides, hydrolyzed or partially hydrolyzed egg shell membrane, fibrin, polylysine, polypropylene imine hexadecylamine, polyethylene imine, glycoproteins, glycosaminoglycans, (e.g., heparan sulfate, chondroitin-4-sulfate, chondroitin-6-sulfate, dermatan sulfate, keratan sulfate).

According to an aspect which is not part of the present invention, the surface of the hydrogel sponge or 3D scaffold can be physically or chemically modified to immobilize substances useful for cell growth, e.g.: collagen, fibronectin, vitronectin, laminin, heparin, plasma, Arg-Gly-Asp (integrin-binding sequence -RGD-) or heparin binding sequence (-FHRRIKA-) peptides, hyaluronic acid, chitosan, chitosan-glycerophosphate, bioactive peptides, polyethylene glycol (PEG) modified with either hydrolysable units or bioactive peptides, fibrin, and glycoproteins, glycosaminoglycans.

According to an aspect which is not part of the invention, the three-dimensional scaffold can be coated with osteogenic proteins such as bone morphogenic protein (BMP) and bone sialoprotein (BSP). BSP, a small integrin-binding ligand N-linked glycoprotein (SIBLING), having osteogenic properties and playing an essential role in bone formation. BSP also mediates mineral deposition, binds type I collagen with high affinity, and binds $\alpha v\beta_3$ and $\alpha v\beta_5$ integrins which mediate cell signaling [37].

The main mineral component of bone tissue is a nonstoichiometric carbonated multi-substituted apatite with calcium to phosphorus ratio (Ca:P) between 1.37 and 1.87

Synthetic hydroxyapatite ($Ca_{10}(PO_4)_6(OH)_2$) is a popular bone replacement material because it has a similar crystal structure (Ca:P ratio fixed at 1.67) to native bone apatite. This resemblance can be exploited to 3D culture bone cells in the scaffolds.

According to an aspect which is not part of the present invention, the scaffolds can be mineralized with other mineralizing components like tricalcium phosphate ($Ca_3(PO_4)_2$), hydroxyapatite ($Ca_5(PO_4)_3OH$), carbonated hydroxya-patite, wollastonite, magnesium silicate, alumina and zirconia. Synthetic hydroxyapatite (HA) or the above-mentioned other mineralizing components can be added to the powder in the first uniform solid mixture in the range between 0.5-70 % or 10-40 % or 20-30% to obtain the compact units which will be submitted to subsequent thermal treatment.

Among the most popular methodologies to mineralize the scaffold there is precipitation of the above-mentioned mineralizing components in the scaffolds [38,39, 40, 41, 42].

It is well known that for hydroxyapatite, the nucleation of mineral nanocrystals takes place in specific loci corresponding to non-collagenous acidic macromolecules, many of which are highly charged from carboxylate groups that can bind $Ca^{2+}$ ions. Following the chemical binding of $Ca^{2+}$, the supersaturation of physiological fluids in phosphate ions and other minor species (e.g. $Na^+$ and $K^+$) provokes the precipitation and nucleation of the mineral phase [43].

In fact, it has been verified that carboxylic acid group (COOH) negatively charged, as those present in the scaffold described in the present invention, are the most potent inducers group of mineralization [44]

According to an aspect which is not part of the invention, the mineralization may be carried out by hydro- and solvo-thermal methods [45 - 54], sol-gel method [55 - 58], or emulsion and microemulsion method [59-61].

The scaffold may also be biomineralized.

Biomineralization refers to the processes by which organisms form minerals; the control exerted by many organisms over mineral formation distinguishes these processes from abiotic mineralization.

The term "biomineralization" is also used to refer to the process of mineralization of scaffolds influenced or favored by the presence of biologic materials, such as intestinal submucosa, collagen, body fluid or simulated body-fluid (SBF). In this case the process can be more precisely defined biomimetic nucleation/mineralization.

SBF's as a rule contain the inorganic ion concentrations of human blood plasma and can be obtained by dissolving reagent grade salts (NaCl, $NaHCO_3$, $Na_2SO_4$, KCl, $K_2HPO_4 \cdot 3H_2O$, $MgCl_2 \cdot 6H_2O$, and $CaCl_2.2H_2O$) in distilled water obtaining a final ionic concentration also more times (e.g. 2x) higher than physiological concentrations. Its pH is adjusted to 7.4 (37 °C) with suitable buffer solution; for example, 0.05 mol L-1) of tris(hydroxymethyl)aminomethane (Tris) and hydrochloric acid.

According to an aspect which is not part of the present invention, the scaffold can be conditioned or soaked in SFB [62-64].

According to an aspect which is not part of the present invention, the skeleton of the scaffold is made of cross-linked polyacrylic acid having free carboxyl groups (-COOH), which can be grafted or linked by covalent or strong and weak ionic bonds or electric bonds with molecules useful for culturing cells, like for example with: peptides (natural or synthetic, as Ac-CGGNGEPRGDTYRAY-NH$_2$ or Ac-CGGFHRRIKA-NH$_2$), proteins, poly(ethylene terephthalate, poly($\epsilon$-caprolactone), carbohydrates (e.g. galactose) and carbohydrate derivatives, and with hyaluronic acid, collagen (all types), Matrigel®, alginate, gelatin, agarose, chitosan, chitosan-glycerophosphate, polyethylene glycol (PEG) with either hydrolysable units in bioactive peptides, bioactive peptides, hydrolyzed egg shell membrane, fibrin and growth factors including, but not limited to, bFGF (Basic fibroblast growth factor), EGF (Epidermal growth factor), VEGF (Endothelial growth factor).

According to an aspect which is not part of the present invention, this scaffold can incorporate nanoparticles within the 3D hydrogel sponge network, either physically or covalently, to embrace various functionalities and properties.

The-above mentioned nanoparticles may be silica, quantum dots, nanotubes, polymers, liposomes, micelles, or dendrimers. Moreover, they may contain different compounds such as, for example, graphene, gold, magnetic iron oxide, tricalcium phosphate ($Ca_3(PO_4)_2$), hydroxyapatite ($Ca_5(PO_4)_3OH$), carbonated hydroxyapatite, wollastonite, mag-nesium silicate, alumina and zirconia.

On the scaffold, according to an aspect which is not part of the present invention, it is possible to grow primary cells, from normal and tumor tissues, immortalized cell lines, embryonic and induced pluripotent stem cells (e.g., ESCs and iPSCs), and mesenchymal stem cells (e.g., MSCs), from fetal and several adult tissues (for example, adipose, bone marrow, skin, liver, kidney, neural tissues).

Such cells include, but are not limited to, endothelial cells, pericytes, macrophages, monocytes, plasma cells, mast cells, adipocytes, hematopoietic tissue and reticular cells, fibroblasts, glial cells, Kupffer cells, vascular and bile duct endothelial cells. Fibroblasts may be derived from organs, such as skin, liver, pancreas, bone marrow, skin, liver, pancreas, kidney, adrenal and neurologic tissue, etc. to name but a few.

A non-limiting list of cells that can be cultured on this 3D scaffold or hydrogel sponge scaffold is reported here and in Table 1.

Such cells include i.e. DU145 (prostate cancer), H295R (adrenocortical cancer), HeLa (cervical cancer), KBM-7 (chronic myelogenous leukemia), LNCaP (prostate cancer), MCF-7 (breast cancer), MDA-MB-468 (breast cancer), PC3 (prostate cancer), SaOS-2 (bone cancer), SH-SY5Y (neuroblastoma, cloned from a myeloma), T47D (breast cancer), THP-1 (acute myeloid leukemia), U87 (glioblastoma), National Cancer Institute's 60 cancer cell line panel (NCI60).

**Table 1: list of cells which may be cultured on a 3D scaffold according to an aspect which is not part of the invention.**

| Cell line | Organism | Origin tissue | Morphology |
|---|---|---|---|
| A172 | Human | Brain | Glioblastoma |
| A253 | Human | Submandibular duct | Head and neck carcinoma |
| A2780 | Human | Ovary | Ovarian carcinoma |
| A2780ADR | Human | Ovary | Adriamycin-re sistant derivative of A2780 |
| A2780cis | Human | Ovary | Cisplatin-resistant derivative of A2780 |
| A431 | Human | Skin epithelium | Squamous cell carcinoma |
| A549 | Human | Lung | Lung carcinoma |
| BCP-1 | Human | PBMC | HIV+ primary effusion lymphoma |
| BEAS-2B | Human | Lung | Epithelial |
| BOSC23 | Human | Kidney (embryonic) | Epithelium |
| BT-20 | Human | Breast epithelium | Breast carcinoma |
| BxPC3 | Human | Pancreatic adenocarcinoma | Epithelial |
| Caco-2 | Human | Colon | Colorectal carcinoma |
| Cal-27 | Human | Tongue | Squamous cell carcinoma |
| CML T1 | Human | CML acute phase | T cell leukemia |
| COR-L23 | Human | Lung | Lung carcinoma |
| COR-L23/5010 | Human | Lung | Lung carcinoma |
| COR-L23/CPR | Human | Lung | Lung carcinoma |
| COR-L23/R23- | Human | Lung | Lung carcinoma |
| COV-434 | Human | Ovary | Ovarian granulosa cell carcinoma |
| DAOY | Human | Brain | Medulloblastoma |
| DU145 | Human | Androgen insensitive prostate carcinoma | |
| DuCaP | Human | Metastatic prostate carcinoma | Epithelial |
| EM-2 | Human | CML blast crisis | Ph+ CML line |
| EM-3 | Human | CML blast crisis | Ph+ CML line |
| FM3 | Human | Lymph node metastasis | Melanoma |
| H1299 | Human | Lung | Lung carcinoma |
| HaCaT | Human | Skin | Keratinocyte |
| HCA2 | Human | Colon | Adenocarcinoma |
| HEK 293 | Human | Kidney (embryonic) | Epithelium |
| HEK 293T | Human | Kidney (embryonic) | Epithelium |

(continued)

| Cell line | Organism | Origin tissue | Morphology |
|---|---|---|---|
| HeLa | Human | Cervix epithelium | Cervical carcinoma |
| Hep G2 | Human | Liver | Hepatoblastoma |
| HL-60 | Human | Blood | Myeloblast |
| HT-1080 | Human | | Fibrosarcoma |
| HT-29 | Human | Colon epithelium | Adenocarcinoma |
| Jurkat | Human | White blood cells | T cell leukemia |
| JY | Human | Lymphoblastoid | EBV-transformed B cell |
| K562 | Human | Lymphoblastoid | CML blast crisis |
| KBM-7 | Human | Lymphoblastoid | CML blast crisis |
| KCL-22 | Human | Lymphoblastoid | CML |
| KG1 | Human | Lymphoblastoid | AML |
| Ku812 | Human | Lymphoblastoid | Erythroleukemia |
| KYO-1 | Human | Lymphoblastoid | CML |
| LNCaP | Human | Prostatic adenocarcinoma | Epithelial |
| Ma-Mel 1, 2, 3....48 | Human | Skin | A range of melanoma cell lines |
| MCF-7 | Human | Breast | Invasive breast ductal carcinoma ER+, PR+ |
| MCF-10A | Human | Breast epithelium | |
| MDA-MB-157 | Human | Pleural effusion metastasis | Breast carcinoma |
| MDA-MB-231 | Human | Pleural effusion metastasis | Breast carcinoma |
| MDA-MB-361 | Human | | Melanoma (contaminated by M14) |
| MDA-MB-468 | Human | Pleural effusion metastasis | Breast carcinoma |
| MG63 | Human | Bone | Osteosarcoma |
| MOR/0.2R | Human | Lung | Lung carcinoma |
| Mono-Mac-6 | Human | White blood cells | Myeloid metaplasic AML |
| MRC-5 | Human | Lung (fetal) | Fibroblast |
| NCI-H69 | Human | Lung | Lung carcinoma |
| NCI-H69/CPR | Human | Lung | Lung carcinoma |
| NCI-H69/LX10 | Human | Lung | Lung carcinoma |
| NCI-H69/LX20 | Human | Lung | Lung carcinoma |
| NCI-H69/LX4 | Human | Lung | Lung carcinoma |
| NALM-1 | Human | Peripheral blood | Blast-crisis CML |
| NK-92 | Human | Leukemia/lymphoma | |
| NTERA-2 | Human | Lung metastasis | Embryonal carcinoma |
| NW-145 | Human | Skin | Melanoma |

(continued)

| Cell line | Organism | Origin tissue | Morphology |
|---|---|---|---|
| OPCN/OPCT cell lines | Human | Prostate | Range of prostate tumour lines |
| PC-3 | Human | Bone metastasis | Prostate carcinoma |
| Peer | Human | T cell leukemia | |
| PNT1A | Human | Prostate | SV40-transformed tumour line |
| PNT2 | Human | Prostate | SV40-transformed tumour line |
| Raji | Human | B lymphoma | Lymphoblast-like |
| SaOS-2 | Human | Bone | Osteosarcoma |
| SH-SY5Y | Human | Bone marrow metastasis | Neuroblastoma |
| SiHa | Human | Cervix epithelium | Cervical carcinoma |
| SK-BR-3 | Human | Breast | Breast carcinoma |
| SK-OV-3 | Human | Ovary | Ovarian carcinoma |
| SK-N-SH | Human | Brain | Epithelial |
| T2 | Human | | T cell leukemia/B cell line hybridoma |
| T-47D | Human | Breast | Breast ductal carcinoma |
| T84 | Human | Lung metastasis | Colorectal carcinoma |
| T98G | Human | Glioblastoma-astrocytoma | Epithelium |
| THP-1 | Human | Monocyte | Acute monocytic leukemia |
| U2OS | Human | Osteosarcoma | Epithelial |
| U373 | Human | Glioblastoma-astrocytoma | Epithelium |
| U87 | Human | Glioblastoma-astrocytoma | Epithelial-like |
| U937 | Human | Leukemic monocytic lymphoma | |
| VCaP | Human | Vertebra metastasis | Prostate carcinoma |
| VG-1 | Human | | Primary effusion lymphoma |
| WM39 | Human | Skin | Melanoma |
| WT-49 | Human | Lymphoblastoid | |
| YAR | Human | Lymphoblastoid | EBV-transformed B cell |
| VERO | African green monkey - Chlorocebus sabaeus | Kidney epithelium | |
| CMT12 | Dog | Mammary gland | Epithelium |
| D17 | Dog | Lung metastasis | Osteosarcoma |
| DH82 | Dog | Histiocytosis | Monocyte/macrophage |
| MDCK II | Dog | Kidney | Epithelium |
| AHL-1 | Hamster | Lung | |
| BHK-21 | Hamster | Kidney | Fibroblast |

(continued)

| Cell line | Organism | Origin tissue | Morphology |
|---|---|---|---|
| CHO | Hamster | Ovary | Epithelium |
| C6/36 | Insect - Asian tiger mosquito | Larval tissue | |
| S2 | Insect - Drosophila melanogaster | Late stage (20-24 hours old) embryos | |
| Sf21 | Insect (moth) - Spodoptera frugiperda | Ovary | |
| Sf9 | Insect (moth) - Spodoptera frugiperda | Ovary | |
| High Five | Insect (moth) - Trichoplusia ni | Ovary | |
| Pt K2 | Long-nosed potoroo - Potorous tridactylus | Kidney | Epithelial |
| 3T3-L1 | Mouse | Embryo | Fibroblast |
| 4T1 | Mouse | Mammary gland | |
| A20 | Mouse | B lymphoma | B lymphocyte |
| ALC | Mouse | Bone marrow | Stroma |
| B16 | Mouse | Melanoma | |
| bEnd.3 | Mouse | Brain/cerebral cortex | Endothelium |
| C2C12 | Mouse | Myoblast | |
| C3H-10T1/2 | Mouse | Embryonic mesenchymal cell line | |
| CGR8 | Mouse | Embryonic stem cells | |
| CT26 | Mouse | Colon | Colorectal carcinoma |
| E14Tg2a | Mouse | | Embryonic stem cells |
| EL4 | Mouse | | T cell leukemia |
| EMT6/AR1 | Mouse | Mammary gland | Epithelial-like |
| EMT6/AR10 . 0 | Mouse | Mammary gland | Epithelial-like |
| GL261 | Mouse | Brain | Glioma |
| Hepalclc7 | Mouse | Hepatoma | Epithelial |
| J558L | Mouse | Myeloma | B lymphocyte cell |
| L1210 | Mouse | Lymphocytic leukemia | Ascitic fluid |
| L243 | Mouse | Hybridoma | Secretes L243 mAb (against HLA-DR) |
| MA2.1 | Mouse | Hybridoma | Secretes MA2.1 mAb (against HLA-A2 and HLA-B17) |
| MC-38 | Mouse | Colon | Adenocarcinoma |
| MTD-1A | Mouse | | Epithelium |
| MyEnd | Mouse | | Endothelium |
| Neuro-2a | Mouse | Nerve/neuroblastoma | Neuronal stem cells |

(continued)

| Cell line | Organism | Origin tissue | Morphology |
|---|---|---|---|
| NIH-3T3 | Mouse | Embryo | Fibroblast |
| Neuro2a | Mouse | Nerve/neuroblastoma | Neuronal stem cells |
| P3X63Ag8 | Mouse | Myeloma | |
| RenCa | Mouse | Kidney | Renal carcinoma |
| RIN-5F | Mouse | Pancreas | |
| RMA-S | Mouse | | T cell tumour |
| YAC-1 | Mouse | Lymphoma | |
| COS-7 | Old World monkey - Cercopithecus aethiops (Chlorocebus) | Kidney | Fibroblast |
| 9L | Rat | Brain | Glioblastoma |
| B35 | Rat | Neuroblastoma | |
| C6 | Rat | Brain astrocyte | Glioma |
| PC12 | Rat | Adrenal medulla | Pheochromocytoma |
| RBL-1 | Rat | Leukemia | Basophil cell |
| OK | Virginia opossum - Didelphis virginiana | Kidney | |
| AB9 | Zebrafish | Fin | Fibroblast |

According to an aspect which is not part of the present invention, the cells were seeded on top of the dried scaffold placed in a culture medium and the culture medium was adjusted within 15-30 minutes. The seeding stage could require a centrifugation step, immediately after overlayering the cells, for 2 to 10 min, not over 1000 g at temperature ranging from 4°C to 37°C to achieve a uniform cell distribution in the scaffold. The different cell cultures were incubated for a different time (up to 60 days) in humidified atmosphere of 5% $CO_2$ and 95% air at 37°C, with a variable medium change frequency depending on cell type, ranging from 1 to 4 days.

According to said aspect which is not part of the present invention, some cellular lines can be cultured into the scaffold under different dynamic conditions by a proper mechanical stimulation which promotes an efficient progenitor cell expansion, or nutrients and catabolites exchange. The scaffold with the cells can be subjected to periodic manual compressive force in a suitable compressive apparatus or device.

To recover the cells, different enzymes can be used, including, but not limited to, trypsin, chymotrypsin, collagenase, elastase, and/or hyaluronidase, DNase, pronase, dispase etc. The cells can be recovered from the scaffold by the application of a mild centrifugation force.

Scaffold mechanical disruption can also be accomplished by a number of methods including, but not limited to, the use of grinders, blenders, sieves, homogenizers, pressure cells or insonators.

The presence or the number or the growth of cells inside the scaffold could be monitored by impedance based assay or by other electrical known measurement or method based on eco-Doppler effect or other acoustic methods.

**Method to produce the three-dimensional scaffold**

According to the present invention, the three-dimensional scaffold is obtained by compression of the cross-linked polyacrilyc acid powder uniformly mixed with at least one water-soluble salt and at least one gas-forming agent. The resulting compact unit is submitted to a thermal treatment and the soluble fraction of the resulting matrix is leached therefrom by repeated washing with suitable amounts of deionized water up to complete swelling of the matrix and disappearance of the soluble fraction.

According to the present invention, the expression "water-soluble fraction" of said unit means the fraction consisting of the at least one water-soluble gas-forming agent and the at least one water-soluble salt, prior to the thermal treatment of the method according to the present invention.

After said thermal treatment, where the at least one gas-forming agent is thermolabile, the expression "water-soluble fraction" of the thermally treated unit means the fraction consisting of the at least one water-soluble salt and the residue resulting from the decomposition of the at least one thermolabile gas-forming agent during the thermal treatment.

Optionally, the scaffold can be frozen and subjected to lyophilization. After conditioning in a suitable volume of water or an aqueous medium, the scaffolds are sterilized by moist heat in autoclave.

The above-mentioned method according to the present invention is explained in great detail below.

**Preparation of the uniform mixed powder**

Since the porosity and pore size are independently controlled by the concentration of the salts, in the initial composition of the mixed powder, and by the particle size range of the salts of the water-soluble fraction, the particles of the salts can be milled and sieved or only sieved or granulated, alone or together, to yield various particle sizes in the range from 50 to 500 $\mu$m.

The thermostable salt and the gas-forming agent can be used as powder aggregates in microbeads or microspheres of different size and shape, as pure components or in combination between them.

Also, the cross-linked polyacrylic acid can be sieved to yield various particle sizes ranging from 50 to 500 $\mu$m.

The powder of each component, before or after sieving, can be dried in oven at 100°C for 30 minutes and the dried powder is stored, before use, in a desiccator containing silica gel.

**Mixing and granulation**

The mixing of the components, each optionally sieved, is conducted in a suitable stainless-steel container using a Turbula® mixer until the mixture of the components is completely uniform, generally in the following manner:

1. a core formed of the 33.3% (w/w) of the final amount of each formulation component is added and mixed at 62 rpm for a variable mixing time depending on the masses at stake (generally 5-60 min);

2. after the set mixing time, another 33.3% (w/w) of the final amount of each formulation component is added and mixed at 62 rpm, for the set mixing time;

3. after the mixing time of the previous step, the remaining amount of each formulation component is added and mixed at 62 rpm for the same mixing time.

Using a suitable mixing jar amounts of mixed powders from 100 mg to 100 kg can be prepared.

Using a different mixing apparatus larger amounts of mixed powders could be prepared

The uniform mixed powder can be directly submitted to a direct compression procedure or can be granulated.

Granulation can be performed in an apparatus for granulation commonly described (Handbook of chemical engineering), through the following preparative steps:

1) spraying pure water (or 25% ethanol aqueous solution) on the uniform mixed powder during the mixing;

2) sieving of the wet mass;

3) drying of the sieved mass at a temperature lower than 70°C.

Alternatively, the granulating fluid can be a saturated solution or brine of the salt used, like NaCl, which can be sprayed on the uniform mixed powder containing the remaining part of the salt used in the formulation.

The granulation can involve only the uniform powder mixture of water soluble salts and the gas forming agents following the above-mentioned procedure and the granulate so obtained, after assaying the concentration of each component of the mixture and optionally sieving, is mixed with the polymeric fraction in a suitable ratio.

The granulation, involving only the water-soluble salts and the gas forming agent, can be performed in spray-dryer or in a fluid bed apparatus.

In spray driers it is possible to spray a solution containing the components of the water-soluble fraction. The solution containing the components of the water-soluble fraction can contain up to 25% (w/w) of ethanol.

The granulate so obtained, after assaying the concentration of each component of the mixture and optionally sieving, is mixed with the polymeric fraction in a suitable ratio.

The expression "polymeric fraction" means at least one cross-linked polyacrylic acid according to the present invention.

In fluid bed a solution containing the water-soluble salts of the water-soluble fraction (e.g. sodium chloride) is sprayed against a fluid bed of the gas-forming agent (e.g. sodium hydrogen carbonate). The solution containing the salt of the water-soluble fraction can contain up to 25% (w/w) of ethanol. The granulate so obtained, after assaying the concentration of each component of the mixture and optionally sieving, is mixed with the polymeric fraction in a suitable ratio.

## Compaction

The powder or the granulate are compacted in a compact unit machine or using a hydraulic press. The shape of the compact unit depends on punch/die shape.

The form of the compressed powder can be any regular three-dimensional geometric shape, and the weight of powder used can be any between 5 mg and 100 kg.

The compression of the uniform powder can be conducted by working with a pressure per gram of powder between 2 kPa/g - 2500 GPa/g or 150 kPa/g - 25 GPa/g or 16 MPa/g -12 GPa/g.

The machine may be a suitably modified model for carrying out multilayer or dry coating tableting.

## Thermal treatment

The treatment is carried out in a precise forced-air oven as the oven of a gas chromatograph and consists of heating to a predetermined treatment temperature and maintaining such temperature for a predetermined time. The treatment temperature ranges from 120 to 160°C (in particular 125-155°C) for a time in the range of 1-120 minutes, as a function of solid mixture weight, in particular 1-20 minutes. The heating rate of the compressed units can vary from 1°C/minute to 50°C/minute.

The temperature program employed is as follows: 0.1 min at 25°C, then heating up to the treatment temperature with a gradient equal to 30°C/min and maintenance of such temperature for the established time, then forced or natural cooling of the compact units to room temperature.

The cooling step after heating can occur naturally or in forced manner, for example controlling the cooling through the ventilation of dry air or inert gas ($N_2$, Ar, He) at room temperature or dry air or inert gas cooled to a temperature lower than room temperature.

After heating, a conditioning time at environmental conditions, which can even last 24 hours, can be necessary. Generally, such waiting time does not affect the quality of the production, but it is in any case preferable to wait a standard time of 5 minutes before the next steps.

After thermal treatment of every compacted unit, the size was measured and the percentage mass loss was calculated ($\Delta m$ %), according to the following equation:

$$\Delta w\% = (w_0 - w)/w_0 * 100$$

where $w_0$ is the initial weight of the compact unit and w is the weight of the same after heat treatment.

## Leaching

The thermally treated compact unit is repeatedly washed in ultra-pure water up to full hydrogel matrix swelling and complete elimination of the water-soluble fraction, evaluated by detecting the water-soluble fraction components, as salt ions, in the washing water.

As the leaching step occurs after the thermal treatment, the water-soluble fraction contains at least one water-soluble salt and the water-soluble residue of at least one thermolabile gas-forming agent.

For example, the presence of chloride ion is measured potentiometrically using a chloride ion selective electrode (ISE) in conjunction with a double-junction reference electrode and a pH meter with an expanded millivolt scale or an ISE meter capable of being calibrated directly in terms of chloride concentration.

Standards and samples are mixed 50:1 with an ionic strength adjustment solution (ISA). Calibration is performed by analyzing a series of standards and plotting mV vs. chloride concentration on semi-log graph or by calibrating the ion meter directly in terms of chloride concentration. The presence of chloride is assayed also by formation of a precipitate with silver nitrate in the washing water acidified by adding diluted nitric acid. The absence of a visible precipitate or opalescence indicates the absence of chloride ions.

Generally, the matrixes are washed with deionized water (leaching liquid) with a volume equal to 2.7 L per g of the compact unit and the washing is repeated replacing for 5-10 times the volume of leaching liquid with fresh water. For each washing the porous hydrogel sponge is kept in water under stirring for a variable time ranging from 15 to 120 min depending on the scaffold weight.

The final point of leaching is determined after the complete matrix swelling when the hydrogel sponge reaches the maximum size and the residual salt ions are no more detectable in washing/leaching water.

For example, the presence of sodium ion is measured by sodium ion selective electrodes.

If necessary, the sodium ion in washing water can be determined potentiometrically using a sodium ion selective electrode (ISE) in conjunction with a double-junction reference electrode, or a chloride combination ISE, and a pH meter with an expanded millivolt scale or an ISE meter capable of being calibrated directly in terms of sodium concentration.

Standards and samples are mixed 50:1 with an ionic strength adjustment solution (ISA). Calibration is performed by analyzing a series of standards and plotting mV vs. sodium concentration on semi-log graph or by calibrating the ion meter directly in terms of sodium concentration.

To evaluate the complete elimination of water-soluble fraction, during the leaching phase, it is possible to compare the weight of the scaffold dried by lyophilization with respect to the nominal amount of polymer. In fact, for a perfect washing out the weight difference should be the lowest.

**Optional step**

According to an aspect of the invention, after the above-mentioned leaching step, the scaffold is stored in a vacuum chamber to increase interconnectivity due to escape of the residual gas produced by the at least one gas-forming agent, which is still trapped in the matrix.

The vacuum conditioning occurs in an evacuable chamber at residual pressure of $100 \times 10^{-3}$ mBar for 10 or 60 min depending on the weight of the conditioned hydrogel sponge.

After vacuum conditioning, a further leaching step may be carried out in order to eliminate the water-soluble residue of the water-soluble fraction, after the escaping of the residual gas.

**Conditioning and cutting**

The leached 3D scaffolds can be conditioned at different pH in different buffers.

For example, they can be conditioned at pH 7.4 in PBS or phosphate buffer saline solution, replacing completely the water or other liquid in the swollen hydrogel sponge obtained after the washing procedure, with this buffer solution. To this aim the swollen hydrogel sponge is conditioned in a suitable volume of PBS (or other buffer), replacing the conditioning buffer with fresh buffer every hour until the hydrogel sponge is completely conditioned in the new solvent/buffer up to constant weight and/or dimensions.

In particular, before sterilization, the matrixes are conditioned in 5 mL of PBS for gram of final weight of completely leached swollen scaffold or hydrogel sponge (i.e. 100 mL per 20 g) for at least 12-20 h, changing the conditioning solution every 1 hour for the initial 6 h and afterwards maintaining the scaffolds in a fresh PBS volume up to 12-20 h, or until maximum diameter contraction or volume contraction.

The replacement of water in the water-swollen hydrogel with PBS solution or other conditioning medium can be obtained by reconstituting the freeze-dried hydrogel sponge or scaffold directly with PBS solution or other conditioning medium.

Conditioning can be carried out also with other solutions or media used in cell culturing, e.g. DMEM or Dulbecco's Modified Eagle Medium, or EMEM (Eagle's Minimal Essential Medium), RPMI (Roswell Park Memorial Institute medium) or other medium used in the field.

Therefore, the scaffold of the invention may be conditioned in water, a buffer solution or cell culturing media.

The porous swollen hydrogel sponge or 3D scaffold can be used as such or cut in a suitable irregular or regular shape: for example, it is possible to obtain a cylinder shape by a cylindrical cut mold or sharp edge tube with a specific internal diameter.

The two previous steps of cutting and conditioning applied to 3D scaffolds can be inverted depending on different subsequent needs.

**Sterilization and lyophilization**

The lyophilization step may occur before or after the sterilization step.

*Sterilization and lyophilization of scaffold conditioned in deionized or ultrapure water or aqueous solutions.*

The swollen matrix conditioned in deionized or ultrapure water or in an aqueous solution from deionized or ultrapure water (e.g. PBS, pH 7.4), in a suitable packaging, may be sterilized by moist heat process in autoclave. In fact, these 3D hydrogel sponge scaffolds resist to the high pressure and temperature of the sterilization treatment by moist-heat in autoclave, maintaining the mechanical and morphological properties and the suitability for culturing cells.

The above-mentioned scaffolds, packaged in sealed pressure-resistant closed vial, can be submitted to a moist heat sterilization procedure, for example heating at 120°C under saturated steam pressure of 98 kPa over atmospheric pressure for 30 minutes, and the sterilized vial can be stored until use.

The scaffold sterilized in PBS solution must be suitably pre-conditioned, with cell culture medium, under aseptic conditions, before culturing the cells.

Then, the sterilized scaffold is frozen at temperatures lower than -25°C and then lyophilized at a pressure lower than $50 \times 10^{-3}$ mBar until complete drying or by a unidirectional freezing procedure, dipping it at a constant rate (from 2 to 10 mm/min) into a cold bath maintained at a constant temperature lower than -100°C, typically at -196°C and then submitted to lyophilization.

*Lyophilization and sterilization of scaffold conditioned in water conditioned in deionized or ultrapure water or aqueous solution*

The swollen scaffolds conditioned in water may be lyophilized as disclosed above.

The lyophilized scaffolds are subjected to a sterilization step, using one of the following well-known methods as, for example, ionizing radiation (gamma and beta), UV exposition, electron accelerator exposition, chemical and gas sterilization or dry heat sterilization. The sterile conditions are necessary to avoid contamination of the cultured cells, not to preserve the scaffold.

**Further steps**

The lyophilized 3D scaffolds obtained according to any one of the sterilizing and leaching steps disclosed above can be stored in in dry form as lyophilized products, packaged in sealed closed vial.

The lyophilized product, stored in sealed vial, can be reconstituted through an aseptic preparative procedure, with water (if the scaffold was conditioned in buffer or salt aqueous solution), buffer (if conditioned in water) or directly with the medium used for cell culturing (if conditioned in water).

The lyophilized product appears as a highly trabecular dry white sponge or a non-transparent solid porous matrix. The scaffold in aqueous storage solution or solvent swells, becoming a transparent and resistant (tough and unerodible) hydrogel sponge, suitable for storage in the storing fluid and under sterile conditions for long time.

**Example 1 Preparation, conditioning and lyophilization**

The materials used in the present invention are summarized in the following Table 2

**Table 2: materials.**

| SUBSTANCE | PRODUCER |
| --- | --- |
| Carbopol 980 NF POLYMER | Lubrizol |
| Carbopol 974P NF POLYMER | Lubrizol |
| Polycarbophil Noveon AA-1 | Lubrizol |
| Sodium chloride | Sigma-Aldrich (>99.5%) |
| Sodium hydrogen carbonate | Merck |
| Disodium hydrogen phosphate dihydrate | Sigma-Aldrich |
| Potassium dihydrogen phosphate anhydrous | Sigma-Aldrich |

**Table 2A: PBS pH 7.4 composition**

| | mM | g/L |
| --- | --- | --- |
| Sodium chloride | 137 | 8.006 |
| Potassium chloride | 2.7 | 0.201 |
| Disodium hydrogen phosphate dihydrate | 8 | 1.424 |
| Potassium dihydrogen phosphate or Monobasic potassium phosphate | 1.5 | 0.204 |

A suitable amount of about 2 g of sodium chloride powder (purity ≥ 99.5%, Sigma Aldrich, US) was milled in high oscillating frequency mill (Mixer Mill MM2, Retsch, D) in a 1.5 mL stainless steel grinding jar containing 3 stainless steel 5 mm-diameter balls at vibrational frequency of 8 Hz for 30 min. The ground NaCl powder was sieved between 250 and 125 μm on ASTM certified test sieves (Giuliani, IT) by an analytical sieve shaker (AS200 Retsch, D).

Carbopol 980 powder (Carbopol 980NF, Lubrizol, US) was sieved between 250 and 125 μm on ASTM certified test sieves (Giuliani, IT).

Sodium hydrogen carbonate (purity ≥ 99.5%, Merck, D) was used as received having a powder granulometry under 250 μm.

**Table 3: initial compositions of some mixed powders used for the compacting unit preparation.**

| code | Carbopol 980 | Sodium hydrogen carbonate | Sodium chloride |
|---|---|---|---|
| C2 | 30 | 30 | 40 |
| C5 | 40 | 30 | 30 |
| C8 | 50 | 30 | 20 |
| C9 | 60 | 30 | 10 |
| C12 | 20 | 30 | 50 |
| C41 | 40 | 20 | 40 |
| C61 | 40 | 40 | 20 |
| code | Carbopol 974P | Sodium hydrogen carbonate | Sodium chloride |
| K2 | 30 | 30 | 40 |
| K8 | 50 | 30 | 20 |
| K61 | 40 | 40 | 20 |
| K5 | 40 | 30 | 30 |
| code | Polycarbophil | Sodium hydrogen carbonate | Sodium chloride |
| P2 | 30 | 30 | 40 |
| P11 | 40 | 10 | 50 |
| P41 | 40 | 20 | 40 |
| P5 | 40 | 30 | 30 |

**Solid mixture preparation**

All powders were mixed in Turbula® mixer at 62 rpm using a stainless-steel jar of suitable volume taking up 60% to 70% of its capacity.

**Table 4: preparation of 10 g of C2 powder.**

| | % (w/w) added | | | |
|---|---|---|---|---|
| **Mixing Steps** | **Carbopol 980** | **Sodium hydrogen carbonate** | **Sodium chloride** | **Mixing time (min)** |
| **I** | 10 | 10 | 13.3 | 20 |
| **II** | 10 | 10 | 13.3 | 40 |
| **III** | 10 | 10 | 13.4 | 60 |

**Table 5: preparation of 10 g of C5 powder.**

| Mixing Steps | % (w/w) added | | | Mixing time (min) |
| | Carbopol 980 | Sodium hydrogen carbonate | Sodium chloride | |
|---|---|---|---|---|
| I | 13.3 | 10 | 10 | 20 |
| II | 13.3 | 10 | 10 | 40 |
| III | 13.4 | 10 | 10 | 60 |

**Table 6: preparation of 10 g of C8 powder.**

| Mixing Steps | % (w/w) added | | | Mixing time (min) |
| | Carbopol 980 | Sodium hydrogen carbonate | Sodium chloride | |
|---|---|---|---|---|
| I | 20 | 20 | 20 | 20 |
| II | 20 | 10 | - | 40 |
| III | 10 | - | - | 60 |

**Compact unit preparation**

300 ± 1 mg of powder were compacted in a 13 mm×1.4 mm (diameter × thickness) evacuable stainless steel die (Perkin-Elmer, US) using a hydraulic press (Specac, UK) applying a force of 1 tons for 15 seconds.

**Compact unit thermal treatment**

The compact units were subjected to thermal treatment positioned on a metal support in a metal meshed basket. The treatment was carried out in the forced-air oven of a gas chromatograph (HP 5890 series II, Perkin Elmer, US). The temperature program employed was as follows: 0.1 min at 25°C, reaching the final temperature of 150°C with a gradient equal to 30°C/min and maintenance of such temperature for 15 min, then the compacted units were cooled to room temperature by forced-air ventilation.

**Scaffold leaching**

The 300 mg compacted units were swollen in 800 mL deionized water (HPLC grade) in a 1 L beaker under stirring; the water was changed every 15 min for at least 7 times until the compacted units were swollen to their maximum size, and until chloride and sodium ions could not be detected any more in the waste water that had pH = 7.

The presence of sodium ion was measured potentiometrically (Compact Titrator G20 Mettlert Toledo, US) using an ion selective electrode (perfectION comb Na$^+$) on 20 mL of washing water, while the presence of Cl$^-$ was assayed by adding 0.5 mL 2M silver nitrate in 10 mL of washing water acidified by adding diluted (1:4) nitric acid. The absence of a visible precipitate indicated the absence of chloride ions.

**PBS conditioning**

Before sterilization the leached scaffold was conditioned in 25 mL of PBS per gram of final weight of completely leached hydrogel sponge (i.e. 100 mL for 20 g) for at least 12-18 h, changing the conditioning solution every hour for the first initial 6 h and afterwards keeping the scaffolds in a fresh PBS volume for 12 h, until reaching the maximum volume contraction.

**Vacuum conditioning (optional step)**

To increase interconnectivity some scaffolds can be submitted to vacuum conditioning in an evacuable chamber at residual pressure of 100×10$^{-3}$ mBar for 10 or 60 min depending on the weight of the conditioned hydrogelsponge.

**Cutting**

The conditioned hydrogel sponge was cut in 9 mm-diameter cylinders by a 9 mm (internal diameter) sharp edge metallic tube.

**Sterilization**

Five 9 mm-diameter cylinders of hydrogel sponge, conditioned in PBS and suspended in 5 mL of PBS, were placed in 10 mL borosilicate glass vial sealed by aluminum crimp cap with PTFE/SIL liner. The crimped vials were sterilized in autoclave at 120°C and saturated steam pressure of 98 kPa over atmospheric pressure for 30 min (Sterilplus Vacum De Lama, IT).

**Lyophilization (optional step)**

The conditioned 3D-scaffolds were frozen at a temperature lower than -25°C, until complete water solidification, and lyophilized at a pressure lower than $50 \times 10^{-3}$ mBar until complete drying. As conclusion of the freeze-drying process, the scaffolds were submitted to a secondary drying by increasing the temperature to 40°C and heating the dried scaffolds, under vacuum conditions, for at least 1 hour. The lyophilization of already sterilized scaffold can be performed in aseptic condition or the lyophilized scaffolds can be submitted to a following energy sterilization procedure (thermal, radiant) or by chemical agents (ethylene oxide, hydrogen peroxide, $ClO_2$ or another sterilizing agent).

**Results**

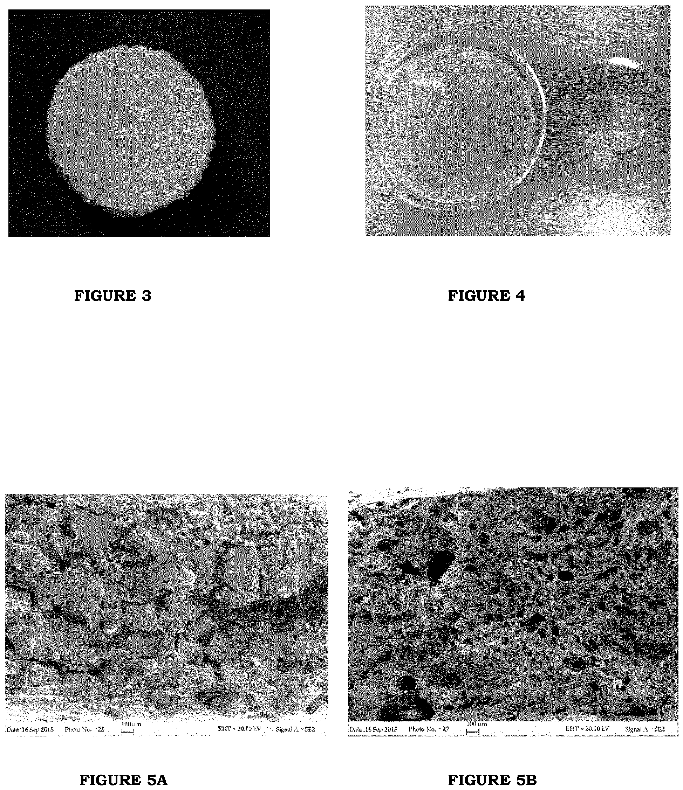

The thermal treatment causes an evident size increase of the compacted unit associated to a weight loss (Tab I-II) while the surface becomes rough (Figs. 2 and 3). The density of the compacted unit decreases of approx. 68%, from 1.74 g/cm$^3$ to 0.56 g/cm$^3$, for C2 and around 85%, from 1.63 to 0.23 g/cm$^3$, for C8 due to the thermal treatment. The leaching in water increases, by swelling, the diameter and thickness of the thermally treated compacted units, more than 3 times for C2 and about 6 times for C8. As shown in Fig. 4, the compacted units not submitted to the thermal treatment (NT), on the right in the photo, disintegrated during the leaching step because the porous matrix was not formed. The sterilization does not cause scaffold weight and size variation with respect to the PBS-conditioned compacted units (p>0.05).

Table 7: morphological and weight change during the preparation of C2 scaffold starting from a 13 × 1.3 mm (diameter × thickness) cylindrical compact unit before cutting.

| weight (mg) | weight change (%) after thermal treatment | diameter (%) after treatment change thermal | thickness change (%) after thermal treatment | SI(%) after leaching | diameter (mm) after PBS conditionin g | thickness (mm) after PBS conditioning | weight (g) after PBS conditioning |
|---|---|---|---|---|---|---|---|
| 300.3 ±0.1 | -12.2±0.4 | 21.6±1.5 | 85.5±1.9 | 5486±569 | 44.6±1.5 | 4.7±0.2 | 8.1±0.4 |

*number of compacted units treated =6*

**26**

**Table 8: morphological and weight change during the preparation of C8 scaffold starting from a 13 × 1.4 mm (diameter × thickness) cylindrical compact unit before cutting.**

| weight (mg) | weight change (%) after thermal treatment | diameter change (%) after thermal treatment | thickness change (%) after thermal treatment | SI(%) after leaching | diameter (mm) after PBS conditionin g | thickness (mm) after PBS conditionin g | weight (g) after PBS conditionin g |
|---|---|---|---|---|---|---|---|
| 300.2 ±0.1 | -12.7±0.9 | 54.5±2.2 | 177.0±10 . 1 | 9878±286 | 64.6±0.7 | 7.6±0.3 | 14.4±1.3 |
| *number of compact units treated =6* | | | | | | | |

## Example 2 <u>Microstructural characterization</u>

Micrographs were acquired by FESEM (Field Emission Scanning Electron Microscope - ZEISS Supra VP40, Carl Zeiss STM, D). The samples were placed on a stub using double-sided conducting tape and coated with a thin Au layer (about 15-20 nm) by sputtering.

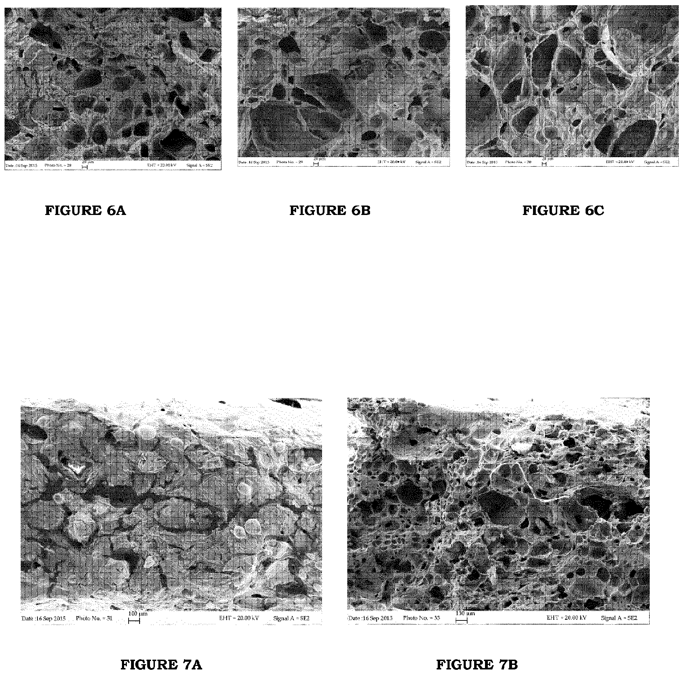

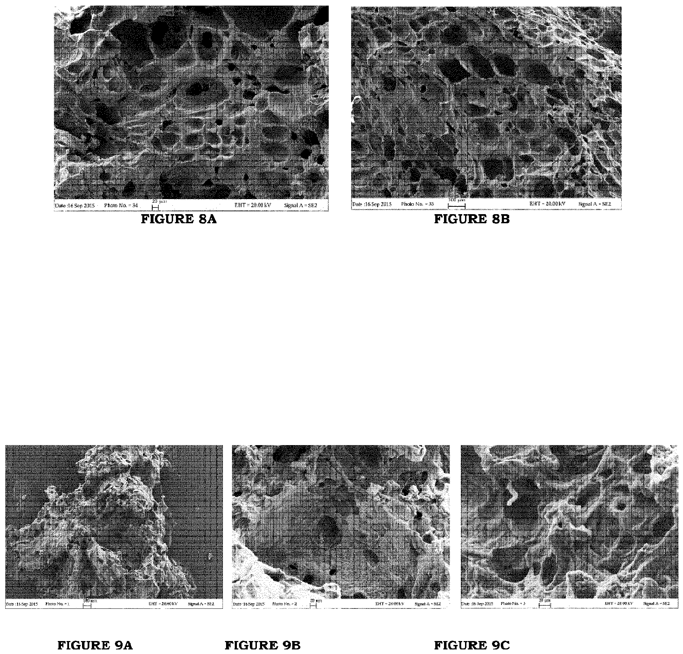

In the first experiment, the SEM micrograph cross sections of the compacted unit of C2 and C5 as such and thermally treated before the leaching procedure were acquired. The comparison of the SEM cross-section of the compacted units as such (left) or thermally treated (right), shown in Figs. 5A, 5B, and 7A and 7B for C2 and C5 scaffolds respectively, highlighted that the controlled heating generates a continuous and porous matrix (zoomed in Figs. 6A-6C and Fig. 8A and 8B for thermally treated C2 and C5 scaffolds, respectively) that allows the swelling of the compacted units in aqueous medium, transforming them into hydrogel sponge scaffolds able, after leaching procedure and suitable conditioning, to support the 3D cell culture. Noteworthy is the behavior of the thermally untreated compacted units when submitted to leaching or conditioning in aqueous medium: they underwent disintegration because did not acquire the heating-induced macroporous architecture, with relevant mechanical and chemical-physical properties, necessary for forming the resistant hydrogel structure useful for vital cellular growth: in fact, in Fig. 4-right the erosion occurred in the untreated matrix during the leaching procedure is evident.

In the second experiment, the SEM micrographs of cross sections of the lyophilized C2 and C5 scaffolds obtained following the previously described procedure, involving the steps of leaching in water, conditioning and sterilization in water or PBS, were acquired.

In the SEM observations, the highly interconnected porous nature of the scaffolds is evident. The distribution and sizes of the pores of the lyophilized scaffold conditioned in PBS (Figs. 7A to 12) is similar to that of the swollen scaffold ready for culturing the cells. The macropore size is up to 100-200 $\mu$m.

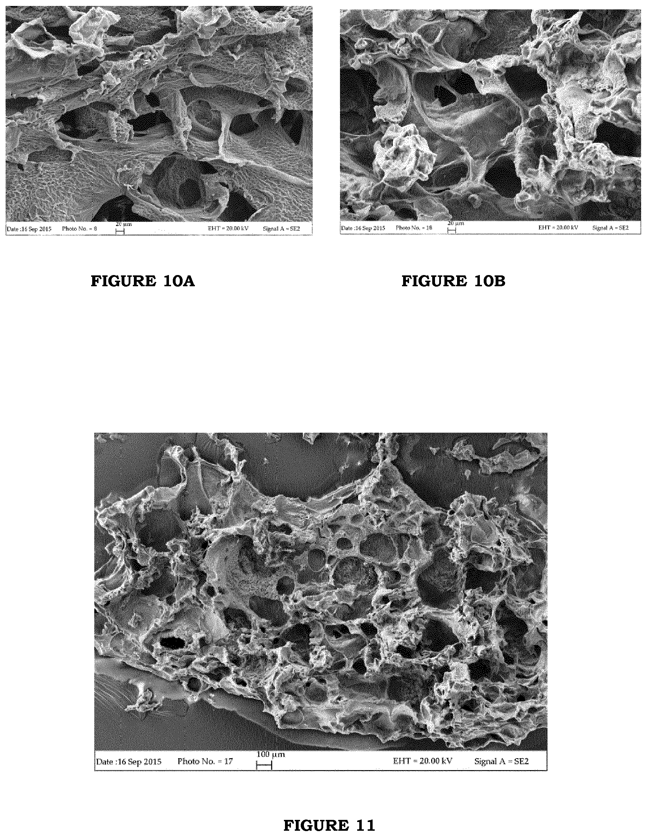

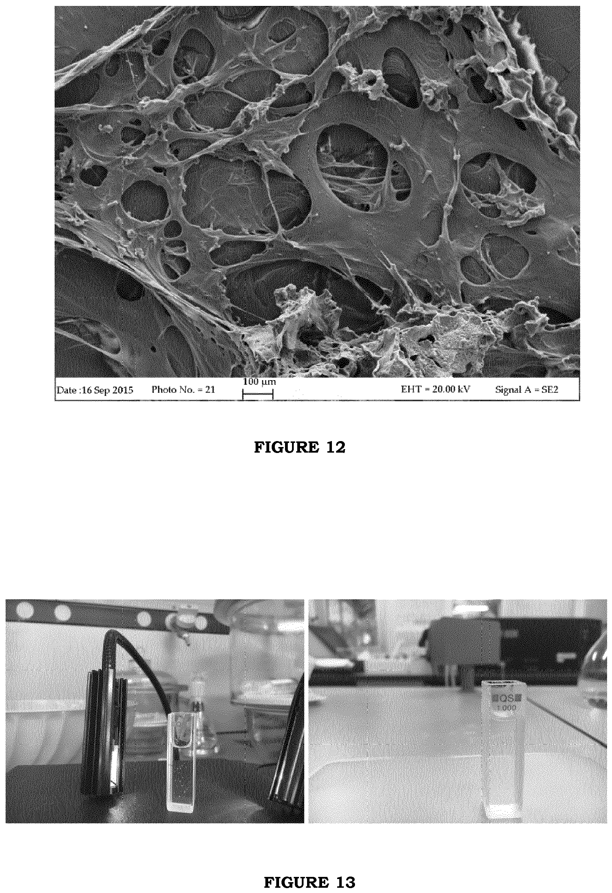

The micrographs of lyophilized scaffolds, after conditioning in water (Fig. 10A, 10-B and 12), clearly show the mechanical tension due to the increased swelling of the hydrogel sponge in this medium with respect to PBS, and consequently the increase of the pore size.

Example 3 <u>Appearance and optically transparency</u>

*Transparency measurement*

It has been necessary to measure the transmittance in the UV-visible spectra also of an unporous matrix obtained by submitting to thermal treatment and PBS conditioning the compacted units composed of the pure cross-linked polyacrilic acid used to prepare the 3D-scaffolds. The water-swollen pure matrixes were cut into cubic portions using a quartz cuvette; for every test, the cuvette was filled with 4 cubes and further PBS was added; the measurements were performed in the visible range (300-800 nm) and the empty cuvette was used as blank. The transmittance was measured using a UV-Visible Spectrometer (HP8452, Hewlett Packard, D)

**Table 9: transmittance (%) values for pure polymer matrix (compacted units, thermally treated, swollen and conditioned) of Carbopol 980 NF, Carbopol 974P NF and Polycarbophil at 400, 600 and 800 nm.**

| Wavelength (nm) | Transmittance (%) | | |
|---|---|---|---|
| | Carbopol 974P NF | Carbopol 980 NF | Polycarbophil |
| 400 | 45 | 43 | 50 |

(continued)

| Wavelength (nm) | Transmittance (%) | | |
|---|---|---|---|
| | Carbopol 974P NF | Carbopol 980 NF | Polycarbophil |
| 600 | 60 | 65 | 65 |
| 800 | 65 | 75 | 70 |

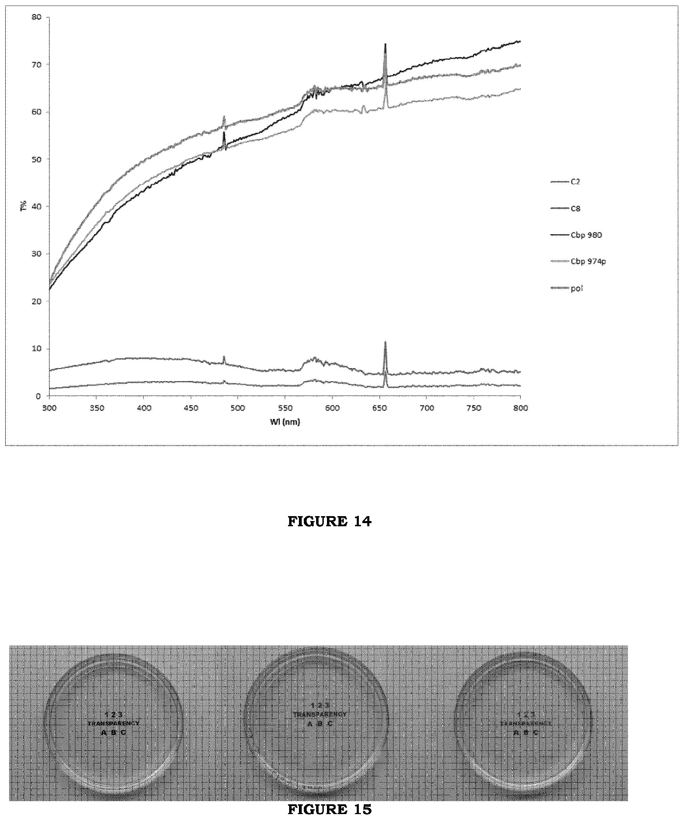

As shown in Fig. 14 the low transmittance of C2 and C8 hydrogel-sponge scaffolds is due to their high porosity, which determines high light reflectivity. The pure polymer matrixes, being non-porous, have higher transmittance (Fig. 13 and Tab. 9). The spectral comparison of C2 and C8 visible spectra with the ones of pure polymer matrixes demonstrates that the visible profile is due to the cross-linked polyacrylic acid used and that the scaffold transmittance is influenced by the interface problem related to the high porosity.

In another experiment, the scaffold transparency was evaluated by observing the letters printed on a paper sheet placed under a Petri capsule containing the PBS-swollen scaffold. The sharpness of the typographical sign, read through the scaffolds completely immersed in PBS, with respect to a reference read through a PBS solution (Fig. 15), proves the good optical properties of the scaffold.

The scaffold transparency is very useful to follow the cell proliferation during cell culture as shown in the micrographs of Figs. from 16 to 18.

## Example 4 Apparent density measurement

The soluble components of PBS-conditioned and sterilized scaffolds (Tab. 3) of cylindrical shape with 15-20 mm diameter were removed by using 800 mL of deionized water, under stirring, replaced every 15 min for at least 7 times until the scaffolds were swollen to their maximum size, and sodium and phosphate ions could be detected any more in the waste water.

The presence of sodium ion is potentiometrically measured (Compact Titrator G20 Mettler Toledo, US) using an ion selective electrode (perfectION comb $Na^+$) on 20 mL of washing water. The absence of phosphate ions in the washing water is tested by adding several mL of ammoniacal solution of ammonium chloride and magnesium chloride: the absence of a visible white precipitate of magnesium ammonium phosphate ($MgNH_4PO_4$) indicates the absence of residual phosphate ions in the washing solution.

The purified scaffolds were frozen at a temperature lower than -18°C until complete water solidification, and lyophilized at pressure lower than $50 \times 10^{-3}$ mBar until complete drying. The dried products were submitted to a secondary drying by heating at 40°C for at least 1 hour, under vacuum. Weight, diameter and thickness of the lyophilized cylindrical scaffolds were measured and the densities were calculated. The density of scaffolds with different compositions is reported in Table 10 4/A (five scaffolds were used for each density measurement); the density was expressed as mean value $\pm$ standard deviation (n=5).

**Table 10: density of scaffolds with different compositions.**

| Scaffold code | Density (mg/cm$^3$) mean $\pm$ SD |
|---|---|
| C2 | 17.6$\pm$0.6 |
| C5 | 20.9$\pm$0.6 |
| C9 | 17.4$\pm$0.7 |
| C12 | 13.7$\pm$0.2 |
| | |
| C41 | 17.5$\pm$1.1 |
| C61 | 17.9$\pm$0.9 |
| K61 | 15.4$\pm$1.1 |
| P41 | 15.2$\pm$1.2 |

## Example 5 Scaffold porosity

Two methods were utilized for measurement of the scaffold porosity [14-18]. The swollen scaffolds, used for porosity measurement, after conditioning and sterilization in PBS, were washed with water up to complete elimination of the soluble fraction and lyophilized following the procedure described in example 4.

Method 1:

For a cylindrical shape lyophilized scaffold, the weight, radius and thickness (or height) were measured and the apparent density was calculated ($\rho_1$), then the scaffolds were cut into smaller pieces, weighed and put into a 13 mm-diameter die and compacted at a compression force of 8 tons for 15 minutes in a hydraulic press under vacuum. Finally, the weight and volume of the produced tablet were measured and its density was calculated ($\rho_2$). The scaffold porosity ($\varepsilon$) was calculated using the following equation:

$$\varepsilon = 1 - \frac{\rho_1}{\rho_2} \times 100$$

Method 2:

The scaffolds were conditioned in acetonitrile because it permeates through the matrix without swelling or shrinking it. The scaffold was placed in acetonitrile and the air bubbles were evacuated under vacuum, until the scaffolds sunk completely in the liquid. The conditioned scaffolds were collected and the excess liquid removed using filter paper and the weight was recorded ($W_w$); the procedure was carried out at room temperature (20 $\pm$ 0.5°C). The recovered scaffolds were dried in forced-air oven at 80°C up to constant weight ($w_s$). The following equation was used to calculate the scaffold porosity ($\varepsilon$):

$$\varepsilon = V_p / (V_p + V_S) \times 100$$

where $V_p$ is the pore volume calculated as follows:

$$V_p = (W_w - W_s) / \rho_{acetonitrile}$$

and $V_s$ the scaffold volume of a 13 mm-diameter cylindrical tablet, obtained by compressing the dried scaffold in a 13 mm-diameter cylindrical die at the force of 8 tons for 15 minutes by hydraulic press eliminating the air using a vacuum pump connected to the die. The volume was measured as:

$$V_S = \pi r^2 h$$

where r is the tablet radius and h is the tablet thickness.
The values of porosity calculated following the two methods are reported in Tab. 11 for some scaffolds of different compositions.

**Table 11: values of porosity calculated following the above-mentioned two methods.**

| Scaffold code | Porosity method 1, mean ± SD (%) | Porosity method 2, mean ± SD (%) |
|---|---|---|
| | | |
| C2 | 97.9±0.1 | 97.6±0.3 |
| C5 | 98.8±0.1 | 97.9±0.3 |
| C8 | 98.7±0.1 | 98.0±0.2 |
| C9 | 98.9±0.1 | 97.8±0.1 |
| C12 | 99.1±0.1 | 98.1±0.3 |
| K61 | 98.2±0.1 | 97.7±0.3 |
| P2 | 97.4±0.1 | 97.2±0.3 |

**Example 6 Compression test**

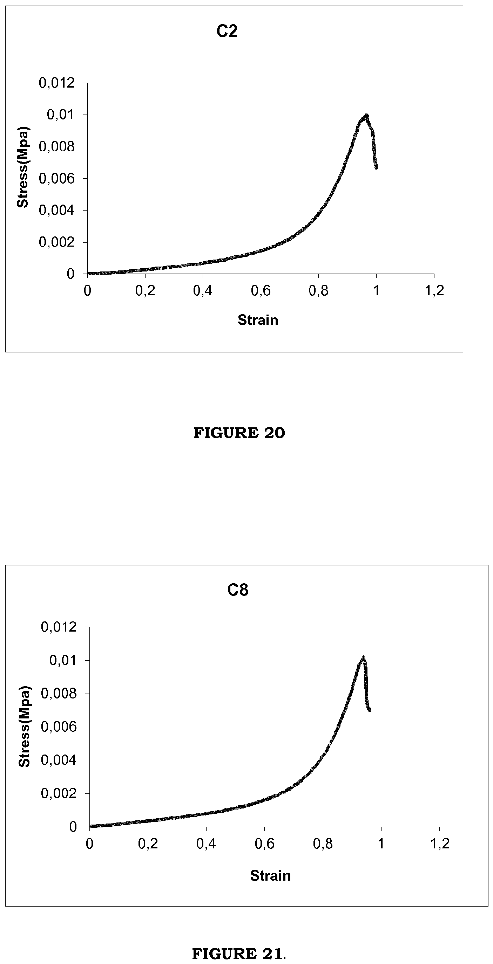

The mechanical properties of the hydrated scaffolds were evaluated by compression tests on LRX dynamometer (Lloyd Instruments, UK), at a speed of 1 mm/min and a temperature of $25\pm2$ °C. Twenty mm-diameter cylindrical scaffolds, conditioned and sterilized in PBS, were submitted to a compression test using a LRX dynamometer equipped with a 20 N load cell and cylindrical (r=5.82 mm) test probe. For the test sterilized and PBS conditioned hydrogel sponge scaffolds were weighed ($w_c$) after removing excess liquid on external surface using filter paper. For the test, sterilized and PBS-conditioned hydrogel sponge scaffolds were weighted ($w_c$) after removing excess liquid on the external surface using filter paper. The tested scaffold was completely immersed in PBS [31] with 1 mm of liquid exceeding the scaffold surface. The probe was moved up to contact with the flat surface of the scaffolds and the elongation was zeroed. The scaffolds were compressed increasing the force (test rate 1 mm/min) up to break. Stress-strain curves were recorded measuring the stress value, referred to the force divided by the area of probe in contact with the scaffold surface, and the strain, referred to the absolute deformation divided by the scaffold thickness. Two stress-strain curves for C2 and C8 scaffolds are shown in Figs. 20 and 21 respectively. The measurements were performed on 6 scaffolds for each composition. The following values have been measured for each scaffold composition:

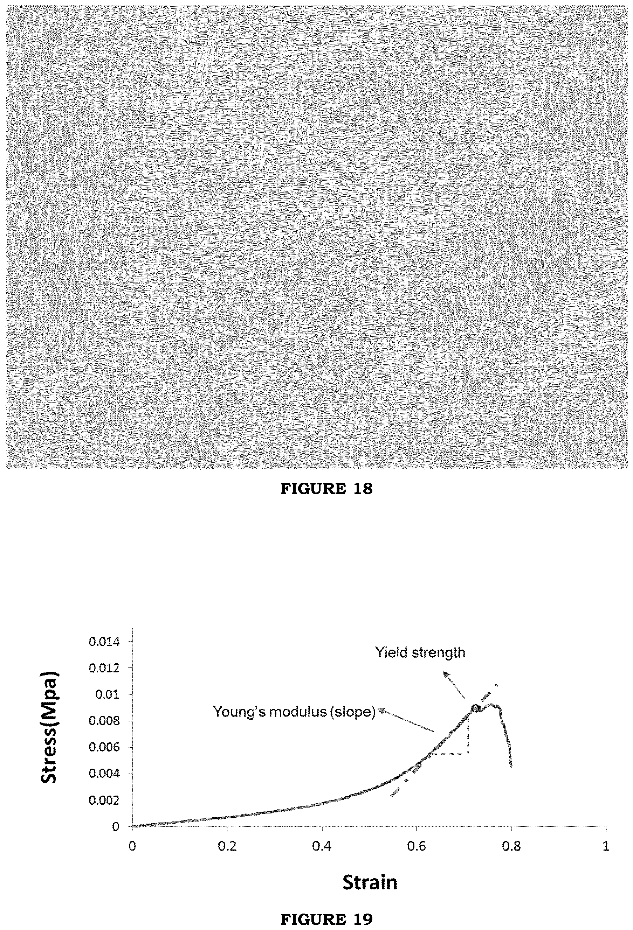

- YM = Young's Modulus (kPa) measured as the slope of the linear part of the stress-strain curve before the plastic deformation at YS (Fig. 19).

- YS = yield strength (Fig. 19), i.e. the stress causing the minimum irreversible deformation in the material, measured as the stress value (kPa);

- Yield strength/weight = yield strength referred to $w_c$, weight of conditioned scaffold.

The YM, YS and Yield strength/weight for some scaffolds are reported in Table 12.

Tab.12: mechanical parameters of different scaffold compositions, reported as

| Scaffold code | PBS conditioned weight (mg) | PBS conditione d Thickness (mm) | Young's modulus (kPa) | Yield strength (kPa) | Yield strength/weig ht (kPa/g) |
|---|---|---|---|---|---|
| C12 | 1221.2 ± 82.5 | 3.71 ± 0.09 | 39.0 ± 2.3 | 7.4 ± 0.7 | 6.46 ± 0.67 |
| C2 | 1456.9 ±1 43.5 | 5.11 ± 0.32 | 38.8 ± 5.2 | 9.8 ± 1.0 | 6.71 ± 0.63 |
| C5 | 1780.2 ± 148.6 | 4.83 ± 0.12 | 52.4 ± 1.9 | 11.7 ± 1.3 | 6.57 ± 0.20 |
| C8 | 1801.3 ± 79.4 | 6.18 ± 0.20 | 53.3 ± 5.9 | 10.3 ± 0.6 | 5.70 ± 0.34 |
| C9 | 2149.1 ± 130.9 | 6.76 ± 0.20 | 68 ± 2.0 | 12.4 ± 0.7 | 5.79 ± 0.49 |
| K5 | 1587.7 ± 22.5 | 5.15±0.1 | 28.3 ± 1.2 | 6.32 ± 0.2 | 3.98 ± 0.09 |
| P5 | 1599.3 ± 51.4 | 5.37 ±0.1 | 38.69 ±2.1 | 7.9 ± 0.3 | 4.97 ± 0.3 |
| mean ± SD (n=6). | | | | | |

**Example 7 Molecular diffusion**

The molecular diffusion through the scaffolds at 37°C was evaluated in horizontal Franz diffusion cell apparatus, using metformin hydrochloride as small, very hydrophilic and soluble diffusing test molecule. The diffusion was studied in PBS-swollen scaffolds with 15 mm diameter and thickness ranging from 3 to 7 mm, which were placed so as to separate the two compartments, fitted into a PTFE ring of 14 mm internal diameter, measuring the resulting increased thickness $(W_H)$ of the hydrogel scaffold. The cell was thermostated at 37°C. The receptor compartment was filled with 21.5 mL PBS solution preheated at 37°C, then the donor compartment was filled with 21.5 mL PBS, containing 500.0 $\mu$g/mL of metformin hydrochloride, preheated at 37°C. Solution aliquots of 500.0 $\mu$L were withdrawn from both compartments at predetermined time periods (at 45 min and 3, 6, 21, 24, 27, 30 h) and metformin hydrochloride concentration was assayed by UV (HP8452 spectrophotometer, Perkin Elmer, US) at λ = 233 nm, against a metformin hydrochloride standard solution (5.0 μg/mL). At any sampling time t, the metformin hydrochloride concentration values in the two chambers were used to calculate the diffusion coefficient D, from the following equation:

$$D = \frac{1}{\beta t} \ln \left( \frac{C_1(t) - C_2(t)}{C_1^0 - C_2^0} \right)$$

with

$$\beta = \frac{A_H}{W_H} \left( \frac{1}{V_1} + \frac{1}{V_2} \right)$$

where:

$C_2^0 = $ initial concentration of metformin hydrochloride in receptor compartment;

$C_1(t)$ = concentration of metformin hydrochloride in donor compartment after time t;

$C_2(t)$ = concentration of metformin hydrochloride in receptor compartment after time t;

$A_H$ = effective cross-sectional area of diffusion in the hydrogel scaffold;

$W_H$ = thickness of the hydrogel scaffold;

$V_1$ = volume of donor compartment;

$V_2$ = volume of receptor compartment.

The diffusion coefficient $D$ was calculated by the slope of ordinary least square regression line obtained plotting $\ln \left( \frac{C_1(t) - C_2(t)}{C_1^0 - C_2^0} \right)$ against time $t$ .

The measured D of some scaffolds are reported in Table 13.

**Table 13: molecular diffusion (D) through some scaffolds at 37°C.**

| Scaffold code | D (37 °C) of metformin hydrochloride (m2/s) (mean ± SD) |
|---|---|
| C2 | $1.06 \times 10^{-9} \pm 8.4 \times 10^{-11}$ |
| C8 | $1.21 \times 10^{-9} \pm 9.6 \times 10^{-11}$ |
| P2 | $1.14 \times 10^{-9} \pm 9.1 \times 10^{-11}$ |
| K8 | $1.19 \times 10^{-9} \pm 9.5 \times 10^{-11}$ |

**Example 8 Microporosity study**

A sample of water conditioned and lyophilized C2 scaffold was submitted to adsorption/desorption measurements ($N_2$ at 77 K) performed with an ASAP 2010 porosimeter (Micromeritics, IT). Pore size distribution was obtained by the well-known BJH method.

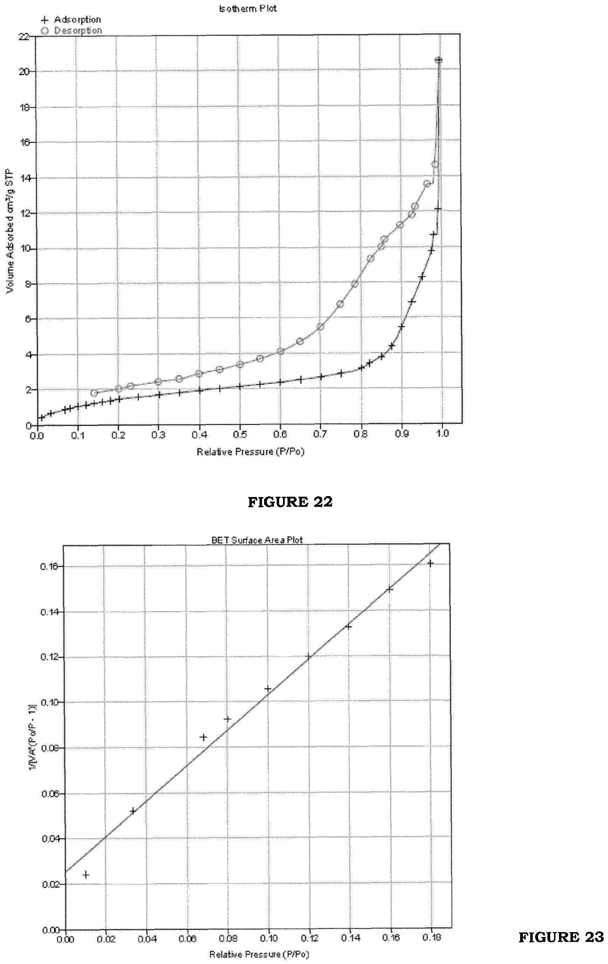

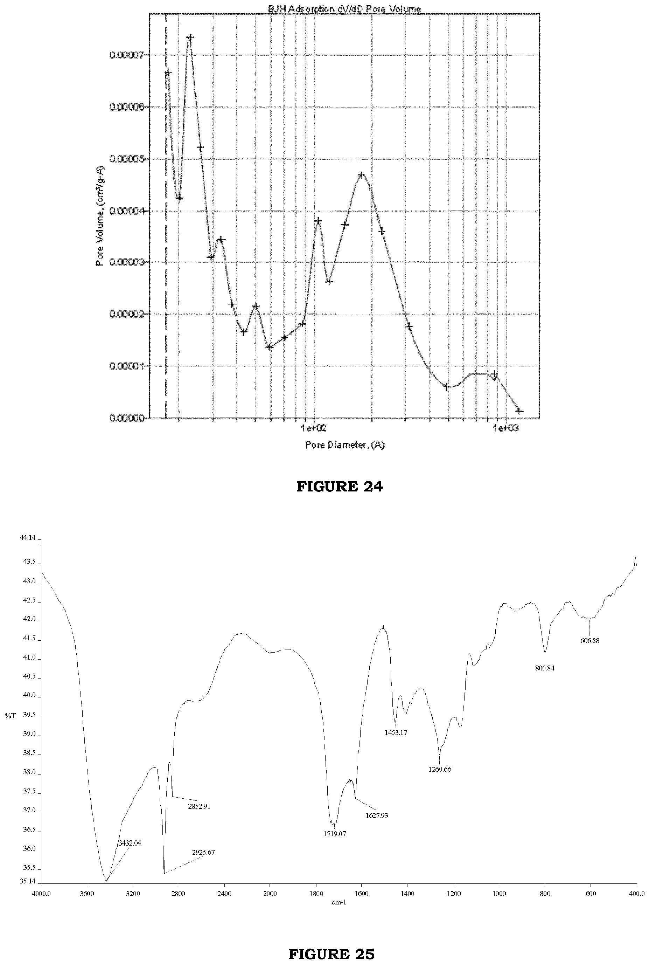

The analysis (Figs. 22, 23) of BET curve indicated the microporous and confirmed the macroporous (see SEM observations of Example 2) nature of the scaffolds. The size distribution of the mesopores is shown in Fig. 24. The BET surface area was 5.4 ± 0.2 m2/g and BJH adsorption cumulative pore volume of pores between 1.7 and 300 nm diameter was 0.017 cm3/g.

**Example 9 IR spectra**

The IR spectra of the different substances and mixtures were acquired through a Perkin Elmer FT-IR 2000 spectrometer (US), preparing the samples in KBr discs.

The IR spectra can be used to characterize the freeze-dried scaffold previously washed in water.

The IR spectra of these products are similar to the ones of the cross-linked polyacrylic components.

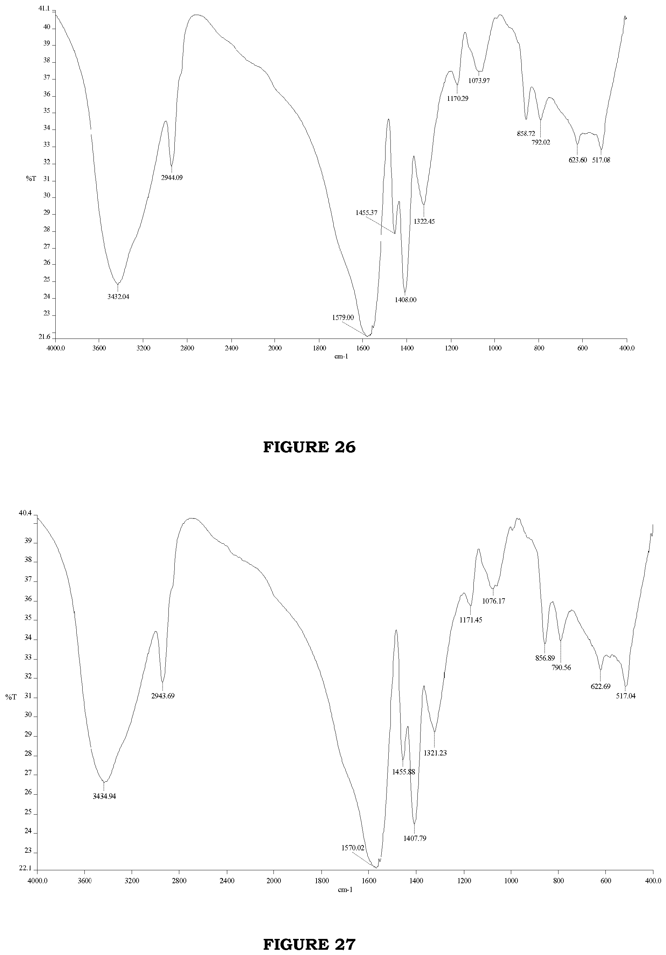

Noteworthy is the presence of C=O stretching band near 1760 cm$^{-1}$ (Fig. 25) for unionized carboxylic acid, typical of the scaffolds with low bicarbonate content; for higher bicarbonate content (Figs. 26 and 27) this band is less evident: a band for asymmetrical stretching of COO$^-$ near 1650-1550 cm$^{-1}$ appears and the band near 1400 cm$^{-1}$ for the symmetrical stretching of COO$^-$ becomes more intense with respect to the band near 1450 cm$^{-1}$. In the presence of only unionized carboxylic group or higher [COOH]/[COO$^-$] ratio, the band near 1450 cm$^{-1}$ becomes more intense with respect to the one near 1400 cm$^{-1}$. The band near 1450 cm$^{-1}$ probably arises from the scissoring vibration of the CH$_2$ group adjacent to the carbonyl and OH bending.

**Example 10 Diffusion test of phenol red into PBS-conditioned scaffold**

**Table 14: morphological characteristics of C2 and C8 hydrogel sponge scaffolds.**

| Scaffold code | Weight (g) | Diameter (mm) | Thickness (mm) |
|---|---|---|---|
| C2 | 0.26±0.02 | 8.5±0.3 | 4.7±0.3 |
| C8 | 0.36±0.02 | 8.3±0.3 | 6.6±0.2 |

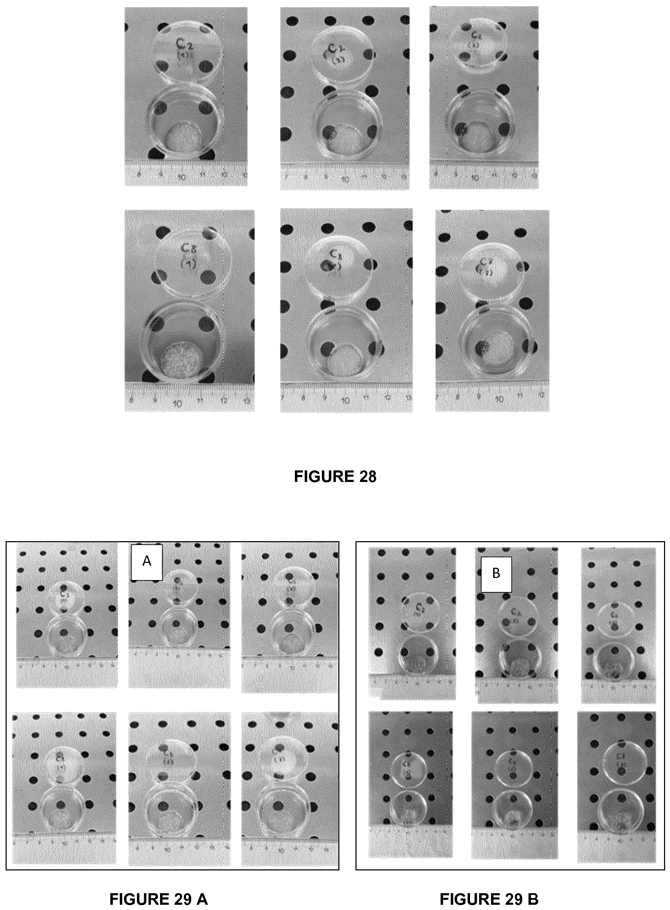

C2 and C8 hydrogel sponge scaffolds, conditioned in PBS, in triplicate, were maintained in PBS solution until use. Then, they were transferred in 12 multiwell plate containing 1 mL of DMEM without phenol red and conditioned overnight to promote the PBS replacement in the gels. The day after, the gels were transferred into Petri dishes containing DMEM added with phenol red and monitored every hour, for the phenol red spread into gel core (Fig. 28).

At the fourth hour, the phenol red was partially spread into the scaffold core (Fig. 29A). Afterwards, the scaffolds were incubated overnight and after 12 hours phenol red was completely spread into the gels (Fig. 29B). The diffusion of phenol red was monitored at 570 nm by TLC Scanner (Camag 3 controlled by WinCATS 1.4.2 software, CH).

**Example 11 Assessment of suitability of hydrogel sponge scaffold for several well-known viability tests: MTT, MTS and NRU**

Where:

MTT is 3-(4,5-dimethylthiazol-2-yl)-2,5-diphenyltetrazolium bromide

MTS is (3-(4,5-dimethylthiazol-2-yl)-5-(3-carboxymethoxyphenyl)-2-(4-sulfophenyl)-2H-tetrazolium)

NR is 3-amino-7-dimethylamino-2-methylphenazine hydrochloride

C2, C8, K2, K8, P2 and P8 hydrogel sponge scaffolds, conditioned in PBS, were maintained in DMEM medium until the assays. To avoid any interference between phenol red present in DMEM and the viability dyes, gels were removed from the plates and were submitted to centrifugation at 720 $\times$ g.

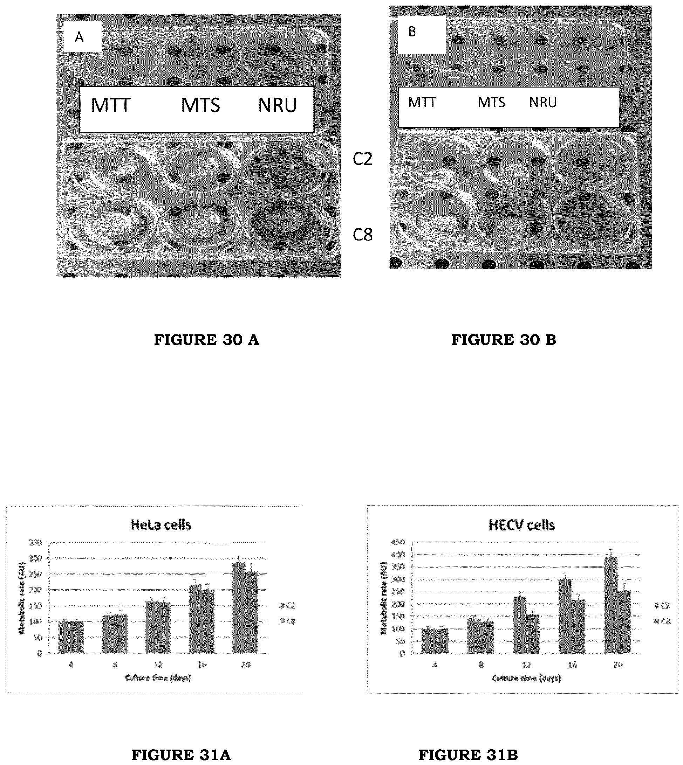

The hydrogel sponge scaffolds were put into a 6 multiwell plate containing 2 mL of MTT, MTS and NRU reagents in duplicate, as shown in Figs. 30A and 30B for C2 and C8 scaffolds.

After a three hour-incubation time, the dyes resulted spread into all gels.

**Example 12 Cell viability**

Cell health can be monitored by numerous methods: plasma membrane integrity, DNA synthesis, DNA content, enzyme activity, presence of ATP, and cellular reducing conditions are known indicators of cell viability and cell death. In this example the wellness of cells was checked by Alamar Blue® assay.

Alamar Blue® cell viability reagent functions as a cell health indicator by using the reducing power of living cells to quantitatively measure the proliferation of various human and animal cell lines, bacteria, plant, and fungi allowing to establish the relative cytotoxicity of agents within various chemical classes. The assay is based on the use of resazurin, a non-toxic, cell-permeable substrate, that is blue and virtually non-fluorescent.

Healthy living cells maintain a reducing state within their cytosol. This "reducing potential" of cells converts resazurin into resorufin, a red highly fluorescent (or absorbent) product. Viable cells continuously convert resazurin to resorufin, increasing the overall fluorescence and color of the media surrounding cells.

Fluorescence of resorufin is detectable in spectrofluorimeter (at excitation/emission wavelengths of 570/585 nm, respectively) as well in spectrophotometer (at 570 nm). In preliminary experiments to assess the optimal time to measure fluorescence following addition of Alamar Blue® in wells, consecutive measurements (1-2-3 hours) by withdrawing 10 μL of experimental medium have been performed. The obtained results suggested that a 3 h incubation time is optimal compared to shorter incubation times

Applied procedures: the different cells studied were seeded into C2, C8, K2, K8, P2 and P8 hydrogel sponge scaffolds conditioned in DMEM, previously reconstituted into single wells of a 24 well plates, at 100 - 200 × 10$^3$ cellular density in 500 μL DMEM medium supplemented with 10% FBS, 2mM glutamine, and were cultured at 37°C in a humidified atmosphere containing 5% $CO_2$.

At each check point time, the scaffolds were transferred (using sterile plastic forceps with flattened tips) into another 24-well plate to perform Alamar Blue® test. Each insert was gently washed with PBS and then 1 mL fresh medium containing 10% Alamar Blue® reagent was added. After incubation, fluorescence was measured (excitation 530 nm, emission 590 nm). Wells containing culture medium without cells and hydrogel, 10% v/v Alamar Blue®, and vitamin C (ascorbic acid 0.75 mg in 5 (μL/well), that results in rapid full reduction of Alamar Blue®, were used as positive controls. Wells with culture medium without cells containing 10% v/v Alamar Blue® were assayed as negative controls.

All the cells tested (A549, Caco-2, Cal-27, HaCaT, HeLa) showed to be healthy when cultured in hydrogel sponge scaffolds (C2, C8, K2, K8, P2 and P8) for a longer time than in 2D culture (at least 20 days), depending on scaffold size and initial density of seeding, since metabolic rate is dependent on cell number present into the scaffold as function of the culture time.

Results for HeLa and HECV cell lines, as models of human epithelial and endothelial cells, are shown for C2 and C8 scaffolds in Figs. 31A and 31B; data are expressed as (AU= Arbitrary Units) extrapolated by fluorescence of resorufin, where metabolic activity registered on 4$^{th}$ day was reported as 100%.

**Example 13 Hydrogel sponge scaffold suitability for test with positive irritation compound (NiSO$_4$)**

The test was performed on HeLa cells seeded into C2 ,C8, K2, K8, P2 and P8 hydrogel sponge scaffolds, after 10 days of culture (seeded at 7.5 × 10$^4$ cells/50 μL). Experimental conditions: exposure of HeLa cells for 3 hours to 0.5 and 5 mM NiSO$_4$, as positive control of irritation. Viability index, expressed as percentage versus the respective untreated culture, was assessed in terms of MTS assay.

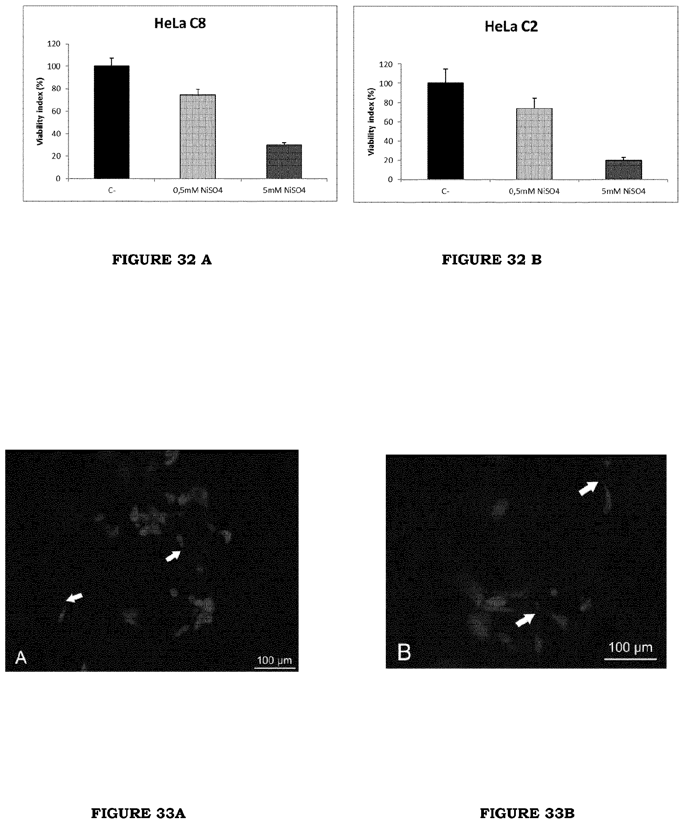

The results evidence the suitability of the hydrogel sponge scaffold for irritation test with nickel sulfate. The test gave similar response for the two kind of scaffolds (Figs. 32A-32B).

**Example 14 Nuclear fluorescent imaging of fixed cells in hydrogel sponge scaffold embedded in agarose**

Preparation of the hydrogel sponge scaffold block and cutting

The hydrogel sponge scaffolds were fixed in paraformaldehyde (PAF) (4% in phosphate buffer saline, PBS 8‰ NaCl and pH 7.4) for 24 h, at 4°C, and then gently washed 3 times with PBS at room temperature in soft agitation. To prepare PAF, 4 g of paraformaldehyde powder were added to 50 mL $H_2Od$, stirring and heating to 56°C until dissolution of the powder. Then, to make the solution (which is initially milky) clear, some drops of NaOH 1 N were added. Finally, 50 mL of PBS (8 % in phosphate buffer saline, PBS 16‰ NaCl and pH 7.4) were added. The solution, cooled down to room temperature, was filtered, aliquoted, and stored at -20°C. The scaffolds were embedded in agarose 3.5% in PBS (3.5 g of agarose powder are dissolved with gentle agitation in PBS, heating up to boiling). The agarose was cooled to 55/56°C and used immediately. The embedded hydrogel sponge scaffolds were maintained in the dark at 4°C and hydrated with PBS until the cutting operation. The cutting was performed in a PBS bath with an appropriate vibratome with a thickness of 40 μm and the sections were immediately mounted on histological slides.

Propidium iodide staining

The following protocol can be used for counter-staining tissue sections or cultured cells on slides.

The staining with propidium iodide (PI) was performed by working solution of PI (1 μg/mL PI and 10 μg/mL RNase A in PBS 8‰ NaCl and pH 7.4), for 40 minutes at room temperature in the dark. After washing many times gently in PBS, the slides were mounted in PBS with cover slip. Glycerol, generally employed in the mounting media, was not used in order to avoid interactions with the hydrogel. The use of RNase A, removing the RNA, allows PI to bind only DNA. PI emits red fluorescence when excited by proper wavelength in epifluorescence microscope or in a CLSM that can excite the PI.

PI stock solution (1 mg/mL - 1.5 mM PI - in deionized water) was stored at 4°C (or aliquoted and stored at -20°C), protected from light. RNase A stock solution (1 mg/mL in bi-distilled water) was aliquoted and stored at -20°C. To prepare the PI working solution (1 $\mu$g/mL PI and 10 $\mu$g/mL RNase A in PBS), 2 $\mu$L of PI stock solution and 20 $\mu$L of RNase A stock solution were added to 2 mL of PBS (8‰ NaCl and pH 7.4). Slides were observed through a Leica DMRB light microscope which was equipped for epifluorescence with a lamp (Mercury Short Arc Photo Optic Lamp HBO 100 W. Osram, D) and proper filter cubes, including A (blue fluorescence), 13 (green fluorescence) and N2.1 (red fluorescence). Micrographs were acquired with a Leica CCD camera DFC420C (Leica, D) and Leica Application Suite (LAS) software. Alternatively, images were obtained using a Leica TCS SL confocal microscope (Leica Microsytems) equipped with argon/He-Ne laser sources, a HCX PL APO CS 63.0×1.40 oil, and HC PL FLUOTAR 20.0×0.5 air objectives.

<u>4',6-diamidino-2-phenylindole, dilactate (DAPI) staining</u>

The following protocol can be used for counter-staining tissue sections or cultured cells on slides.

The staining with DAPI was performed using a working solution of DAPI (100 ng/mL or 300 nM in PBS 8‰ NaCl and pH 7.4) and with an incubation of the sections in dark for 10 minutes at room temperature. Without washing, the sections were mounted with cover slip and PBS as reported above. DAPI binds only DNA by intercalation and, only when bound, it emits blue fluorescence when excited by proper wavelength in epifluorescence microscope, or in a CLSM that can excite the DAPI.

DAPI stock solution (5 mg/mL or 14.3 mM) (using DAPI Molecular Probes, Cat# D-1306) was prepared as follows: 10 mg DAPI added to 1 mL of dimethyl-sulfoxide then to 9 mL of ethyl alcohol absolute and mixed to complete dissolution, aliquoted, and stored at -20°C. DAPI working solution (100 ng/mL or 300 nM in PBS) was obtained diluting 2 $\mu$L of DAPI stock solution in 100 mL of PBS, and stored at 4 °C in dark bottle. Observations were performed with an epifluorescence microscope as reported above.

**Results**

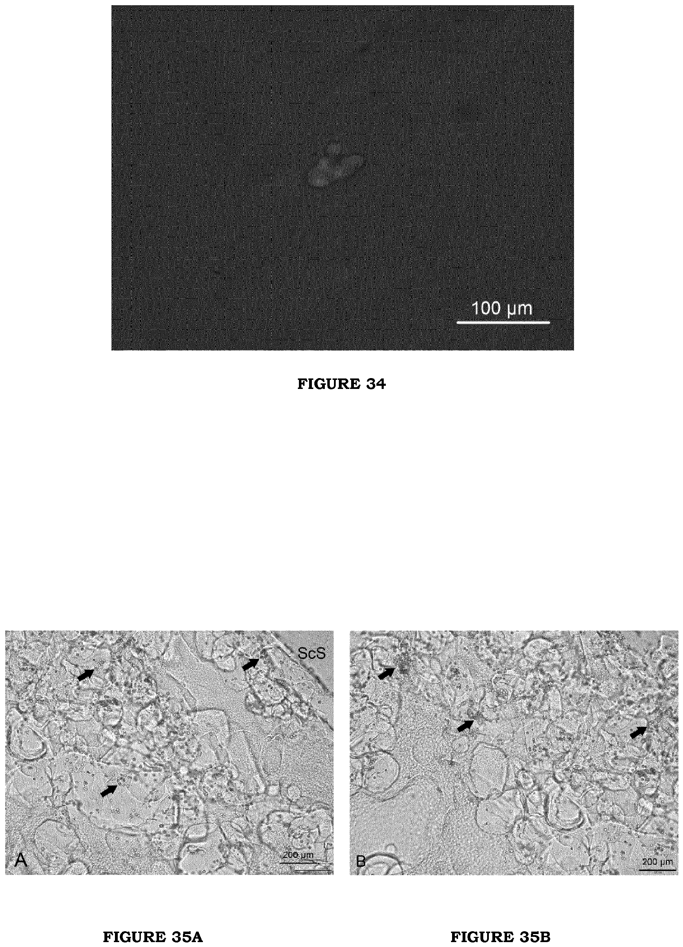

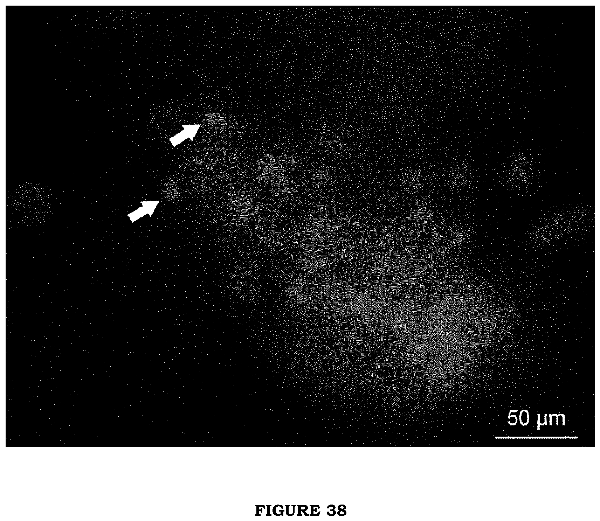

The scaffolds are colonized by all cultured cells from the seeding surface to the bottom, as shown for C2 (Figs. 33A-33B) and C8 (Figs. from 34 to 38) scaffolds. Ordered cellular structures, similar to spheroids, for HECV (Fig. 34) and for HeLa (Fig. 38) are evident.

**References**

1. Lee J, Cuddihy MJ, Kotov NA: Three-dimensional cell culture matrices: State of the art. Tissue Engineering Part B-Reviews 2008, 14(1):61-86.

2. Andrei G: Three-dimensional culture models for human viral diseases and antiviral drug development. Antiviral Research 2006, 71(2-3):96-107.

3. Ravi M, Paramesh V, Kaviya SR, Anuradha E, Solomon FDP: 3D Cell Culture Systems: Advantages and Applications. Journal Of Cellular Physiology 2015, 230(1): 16-26.

4. Xu X, Farach-Carson MC, Jia X: Three-dimensional in vitro tumor models for cancer research and drug evaluation. Biotechnology Advances 2014, 32(7): 1256-1268.

5. Smith SJ, Wilson M, Ward JH, Rahman CV, Peet AC, Macarthur DC, Rose FRAJ, Grundy RG, Rahman R: Recapitulation of Tumor Heterogeneity and Molecular Signatures in a 3D Brain Cancer Model with Decreased Sensitivity to Histone Deacetylase Inhibition. Plos One 2012, 7(12).

6. Picollet-D'hahan N, Dolega ME, Liguori L, Marquette C, Le Gac S, Gidro X, Martin DK: A 3D Toolbox to Enhance Physiological Relevance of Human Tissue Models. Trends in Biotechnology 2016, 34(9):757-769.

7. Chuah JKC, Zink D: Stem cell-derived kidney cells and organoids: Recent breakthroughs and emerging applications. Biotechnology Advances 2017, 35(2): 150-167.

8. Prestwich GD: Evaluating drug efficacy and toxicology in three dimensions: Using synthetic extracellular matrices in drug discovery. Accounts of Chemical Research 2008, 41(1): 139-148.

9. Yang J, Shi GX, Bei JZ, Wang SG, Cao YL, Shang QX, Yang GG, Wang WJ: Fabrication and surface modification of macroporous poly(L-lactic acid) and poly(L-lactic-co-glycolic acid) (70/30) cell scaffolds for human skin fibroblast cell culture. Journal of Biomedical Materials Research 2002, 62(3):438-446.

10. Klein F, Richter B, Striebel T, Franz CM, von Freymann G, Wegener M, Bastmeyer M: Two-Component Polymer Scaffolds for Controlled Three-Dimensional Cell Culture. Advanced Materials 2011, 23(11): 1341-1345.

11. Nie L, Chen D, Suo JP, Zou P, Feng SB, Yang Q, Yang SH, Ye SN: Physicochemical characterization and biocompatibility in vitro of biphasic calcium phosphate/polyvinyl alcohol scaffolds prepared by freeze-drying method for bone tissue engineering applications. Colloids and Surfaces B-Biointerfaces 2012, 100:169-176.

12. Bokhari M, Carnachan RJ, Przyborski SA, Cameron NR: Emulsion-templated porous polymers as scaffolds for three dimensional cell culture: effect of synthesis parameters on scaffold formation and homogeneity. J Mater Chem 2007, 17(38):4088-4094.

13. Ruedinger F, Lavrentieva A, Blume C, Pepelanova I, Scheper T: Hydrogels for 3D mammalian cell culture: a starting guide for laboratory practice. Applied Microbiology and Biotechnology 2015, 99(2):623-636.

14. Rouquerol J, Avnir D, Fairbridge CW, Everett DH, Haynes JH, Pernicone N, Ramsay JDF, Sing KSW, Unger KK: Recommendations For The Characterization Of Porous Solids. Pure and Applied Chemistry 1994, 66(8):1739-1758.

15. Caliari SR, Burdick JA: A practical guide to hydrogels for cell culture. Nat Methods 2016, 13(5):405-414.

16. Zhang D, Pekkanen-Mattila M, Shahsavani MO, Falk A, Teixeira AI, Herland A: A 3D Alzheimer's disease culture model and the induction of P21-activated kinase mediated sensing in iPSC derived neurons. Biomaterials 2014, 35(5):1420-1428.

17. Wu X, Liu Y, Li X, Wen P, Zhang Y, Long Y, Wang X, Guo Y, Xing F, Gao J: Preparation of aligned porous gelatin scaffolds by unidirectional freeze-drying method. Acta Biomaterialia 2010, 6(3):1167-1177.

18. Annabi N, Nichol JW, Zhong X, Ji CD, Koshy S, Khademhosseini A, Dehghani F: Controlling the Porosity and Microarchitecture of Hydrogels for Tissue Engineering. Tissue Engineering Part B-Reviews 2010, 16(4):371-383.

19. Nam YS, Yoon JJ, Park TG: A novel fabrication method of macroporous biodegradable polymer scaffolds using gas foaming salt as a porogen additive. Journal ofBiomedical Materials Research 2000, 53(1): 1-7.

20. Nieto-Suarez M, Lopez-Quintela MA, Lazzari M: Preparation and characterization of cross-linked chitosan/gelatin scaffolds by ice segregation induced self-assembly. Carbohydr Polym 2016, 141:175-183.

21. Botchwey EA, Pollack SR, Levine EM, Laurencin CT: Bone tissue engineering in a rotating bioreactor using a microcarrier matrix system. Journal of Biomedical Materials Research 2001, 55(2):242-253.

22. Teng WQ, Long TJ, Zhang QR, Yao K, Shen TT, Ratner BD: A tough, precision-porous hydrogel scaffold: Ophthalmologic applications. Biomaterials 2014, 35(32):8916-8926.

23. Ostrowska-Czubenko J, Gierszewska-Druzynska M: Effect of ionic crosslinking on the water state in hydrogel chitosan membranes. Carbohydr Polym 2009, 77(3):590-598.

24. Hopkins AM, De Laporte L, Tortelli F, Spedden E, Staii C, Atherton TJ, Hubbell JA, Kaplan DL: Silk Hydrogels as Soft Substrates for Neural Tissue Engineering. Adv FunctMater 2013, 23(41):5140-5149.

25. Hayward AS, Sano N, Przyborski SA, Cameron NR: Acrylic-Acid-Functionalized PolyHIPE Scaffolds for Use in 3D Cell Culture. Macromol Rapid Commun 2013, 34(23-24):1844-1849.

26. Son JS, Park K, Oh S, Kim J-J, Han DK: Preparation and characterization of porous heparin-immobilized PLGA scaffolds using novel solid hydrogen peroxide for cartilage regeneration. Tissue Eng Regen Med 2008, 5(3):528-534.

27. Purchio, A. F.; Zimber, M.; Dunkelman, N.; Naughton, G. K.; Naughton, B. A. Three-dimensional cartilage cultures using transforming growth factor-β. US5902741A, 1999.

28. Liu, G. M. Methods and compositions for growing corneal endothelial and related cells on biopolymers and creation of artificial corneal transplants. WO2005037144A2, 2005.

29. Sheetz, M. P.; Hone, J. C.; Tanase, M.; Wind, S. J. Active cell growth substrates and apparatus for applying perturbation to a cell in vitro. WO2007103135A2, 2007.

30. Liu, Q. Three dimensional cell culture construct and apparatus for its making. US20080194010A1, 2008.

31. Shin, J. U.; Shin, J. W.; Park, S. H. Complex support body comprising biodegradable polymers, ceramics, and precursor cells for treating bone-cartilage diseases. KR2012111380A, 2012.

32. Zussman, E.; Chaganti, S. R.; Venugopal, J. R.; Ramakrishna, S.; Regev, O. Fiber-reinforced hydrogel composites and methods of forming fiber-reinforced hydrogel composites. WO2013172788A1, 2013.

33. Yue, Z.; Wen, F.; Yu, H. Forming porous scaffold from cellulose derivatives. US8283028B2, 2012.

34. Zhang J, Yang Z, Li C, Dou Y, Li Y, Thote T, Wang D-a, Ge Z: Cells Behave Distinctly Within Sponges and Hydrogels Due to Differences of Internal Structure. Tissue Engineering Part A 2013, 19(19-20):2166-2175.

35. Bogdanowicz DR, Lu HH: Multifunction Co-culture Model for Evaluating Cell-Cell Interactions. In: Biomimetics and Stem Cells: Methods and Protocols. Edited by Vunjak-Novakovic G, Turksen K. New York, NY: Springer New York: 29-36.

36. D.P. Mohanty, S. Mohapatra, S. Misra, P.S. Sahub Milk derived bioactive peptides and their impact on human health - A review, Saudi J Biol Sci. 2016 Sep; 23(5): 577-583.

37. Kruger, T. E.; Miller, A. H.; Wang, J. X., Collagen Scaffolds in Bone Sialoprotein-Mediated Bone Regeneration. Sci. World J. 2013.

38. Ferraz, M.P., F.J. Monteiro, and C.M. Manuel, Hydroxyapatite nanoparticles: A review of preparation methodologies. J Appl Biomater Biomech, 2004. 2(2): p. 74-80.

39. Cunniffe, G.M., et al., The synthesis and characterization of nanophase hydroxyapatite using a novel dispersant-aided precipitation method. Journal of Biomedical Materials Research Part A, 2010. 95A(4): p. 1142-1149.

40. Wang, P.P., et al., Effects of synthesis conditions on the morphology of hydroxyapatite nanoparticles produced by wet chemical process. Powder Technology, 2010. 203(2): p. 315-321.

41.Bouyer, E., F. Gitzhofer, and M.I. Boulos, Morphological study of hydroxyapatite nanocrystal Suspension. Journal of Materials Science-Materials in Medicine,2000. 11(8): p. 523-531.

42. Kothapalli, C., et al., Influence of temperature and concentration on the sintering behavior and mechanical properties of hydroxyapatite. Acta Materialia, 2004. 52(19): p. 5655-5663.

43.Tampieri, A.; Sprio, S.; Sandri, M.; Valentini, F., Mimicking natural bio-mineralization processes: A new tool for osteochondral scaffold development. Trends in Biotechnology 2011, 29 (10), 526-535.

44. Kretlow, J. D.; Mikos, A. G., Review: Mineralization of synthetic polymer scaffolds for bone tissue engineering. Tissue Engineering 2007, 13 (5), 927-938.

45. Niu, J.L., Hydrothermal synthesis of nano-crystalline hydroxyapatite. Bioceramics, Vol 19, Pts 1 and 2, 2007. 330-332: p. 247-250.

46.Yoshimura, M. and K. Byrappa, Hydrothermal processing of materials: past, present and future. Journal of Materials Science, 2008. 43(7): p. 2085-2103.

47. Manafi, S.A., et al., Synthesis of nano-hydroxyapatite under a sonochemical/hydrothermal condition. Biomedical Materials, 2008. 3(2).

48. Chaudhry, A.A., et al., Instant nano-hydroxyapatite: a continuous and rapid hydrothermal synthesis. Chemical Communications, 2006(21): p. 2286-2288.

49. Manafi, S.A., et al., Synthesis of nano-hydroxyapatite under a sonochemical/hydrothermal condition. Biomedical Materials, 2008. 3(2).

50. Guo, X.Y. and P. Xiao, Effects of solvents on properties of nanocrystalline hydroxyapatite produced from hydrothermal process. Journal of the European Ceramic Society, 2006. 26(15): p. 3383-3391.

51. Wang, Y.J., et al., Investigations on the formation mechanism of hydroxyapatite synthesized by the solvothermal method. Nanotechnology, 2006. 17(17): p.4405-4412.

52. Zhang, S.Y., et al., Kinetic studies on the synthesis of hydroxyapatite nanowires by solvothermal methods. Australian Journal of Chemistry, 2007. 60(2): p. 99-104.

53. Ma, M.G. and J.F. Zhu, Solvothermal Synthesis and Characterization of Hierarchically Nanostructured Hydroxyapatite Hollow Spheres. European Journal of Inorganic Chemistry, 2009(36): p. 5522-5526.

54. Yang, C., et al., Solvothermal synthesis and characterization of Ln (Eu(3+),Tb(3+)) doped hydroxyapatite. Journal of Colloid and Interface Science, 2008. 328(1): p. 203-210.

55. Liu, D.M., T. Troczynski, and W.J. Tseng, Water-based sol-gel synthesis of hydroxyapatite: process development. Biomaterials, 2001. 22(13): p. 1721-1730.

56. Bezzi, G., et al., A novel sol-gel technique for hydroxyapatite preparation.Materials Chemistry and Physics, 2003. 78(3): p. 816-824.

57. Weng, W.J., et al., The effect of citric acid addition on the formation of sol-gel derived hydroxyapatite. Materials Chemistry and Physics, 2002. 74(1): p. 92-97.

58. Agrawal, K., et al., Synthesis of HA by various sol-gel techniques and their comparison: a review. National Conference on Advancements and Futuristic Trends in Mechanical and Materials Engineering, 2011.

59. Zhang, H. And Cooper, A. I., Synthesis and applications of emulsion-templated porous materials. Soft Matter, 2005. 1: p. 107-113

60. Lim, G. K., et al., Processing of hydroxyapatite via microemulsion and emulsion routes. Biometerials, 1997. 18: p. 1433 - 1439.

61. Yang, J. H., et al., Synthesis of spherical hydroxyapatite granules with interconnected pore channels using camphene emulsion. J Biomedical Materials Research Part B, 2011. 99B(1): p. 150-157.

62.Yang et al. Biomineralization of Natural Collagenous Nanofibrous Membranes and Their Potential Use in Bone Tissue Engineering J Biomed Nanotechnol. 2015. 11(3): p. 447-456.

63.Pawelec et al. Biomineralization of Recombinant Peptide Scaffolds: Interplay among Chemistry, Architecture, and Mechanics ACS Biomater. Sci. Eng., 2017. 3(6): p 1100-1108

64. Xu et al. Biomimetic mineralization J. Mater. Chem., 2007. 17: p. 415-449

## Claims

1. A method for the preparation of a three-dimensional hydrogel scaffold for cell culturing, which comprises the steps of:

a) preparing at least one first uniform solid mixture comprising at least one gas-forming agent, selected from the group consisting of sodium hydrogen carbonate, potassium hydrogen carbonate, sodium glycine carbonate, ammonium hydrogen carbonate, sodium bisulfite, sodium metabisulfite, calcium carbonate and sodium sulfite, at least one water-soluble salt and at least one non-water-soluble, water-swellable cross-linked poly(acrylic) polymer;

b) preparing compressed or compact units starting from said at least one first solid mixture;

c) subjecting the compressed or compact units thus obtained to heating at a temperature in the range of 120°C-160°C, for a time of 1-120 minutes, preferably 1-20 minutes;

d) leaching the thermally treated units thus obtained in an aqueous medium;

e) conditioning the leached units thus obtained in water or in an aqueous solution selected from the group comprising a buffer solution, preferably phosphate-buffered saline (PBS) and a cell culture medium;

f) subjecting the conditioned units thus obtained to sterilization.

2. The method according to claim 1, wherein the sterilized units obtained in said step f) are subjected to lyophilization.

3. The method according to claim 1 or 2, wherein said compressed or compact units are prepared by direct compression of said solid mixture, in which said at least one gas-forming agent, said at least one water-soluble salt and said at least one non-water-soluble, water-swellable cross-linked poly(acrylic) polymer are all in powder form.

4. The method according to claim 1 or 2, wherein said compressed or compact units are prepared by compressing said solid mixture, preferably at a pressure comprised between 2 kPa/g and 2500 GPa/g, more preferably 150 kPa/g to 25 GPa/g, for a period ranging from 0.1 seconds to 30 minutes, in which said at least one non-water-soluble, water-swellable cross-linked poly(acrylic) polymer is in powder form and said at least one gas-forming agent and said at least one water-soluble salt have previously been granulated or spheronized.

5. The method according to any one of claims 1 to 4, which comprises

- preparing a second uniform solid mixture comprising at least one non-water-soluble, water-swellable cross-linked poly(acrylic) polymer, which can be the same as said first uniform solid mixture or different therefrom;
- forming a layer of said second uniform solid mixture, depositing a layer of said first solid mixture over said layer of second uniform solid mixture and optionally depositing one more layer of said second uniform solid mixture onto said layer of first solid mixture;

wherein said compressed or compact units of step b) are prepared by compressing all of said layers.

6. The method according to any one of claims 1 to 4, which comprises

- preparing a second uniform solid mixture comprising at least one non-water-soluble, water-swellable cross-linked poly(acrylic) polymer, which can be the same as said first uniform solid mixture or different therefrom;
- preparing a compressed or compact core from said first uniform solid mixture;
- forming a layer of said second uniform solid mixture inside a mold, having a bottom and a peripheral wall, depositing said compressed or compact core onto said layer, the compressed or compact core being less wide than the mold, so that a gap is formed between the core and the peripheral wall,
- filling said gap with said second uniform solid mixture, until the upper surface of said second uniform solid mixture is flush with the upper surface of said compressed or compact core;

wherein said compressed or compact units of step b) are prepared by compressing said second uniform solid mixture and said compressed or compact core contained in said mold.

7. The method according to any one of claims 1 to 6, wherein the compressed or compact units obtained in step b) are partially coated with a film made of a polymeric material selected from the group consisting of hydroxypropylcellulose (HPC), ethylcellulose (EC), hydroxyethylmethylcellulose (HEMC), hydroxypropylmethylcellulose (HPMC), methylcellulose (MC), polyvinylalcohol (PVA), polyvinylalcohol acetate phthalate, polymethacrylates (i.e. Eudragit) and mixtures thereof.

8. The method according to any one of claims 1 to 7, wherein after said step d) of leaching, the leached units are kept at a pressure comprised between $80 \times 10^{-3}$ mBar and $120 \times 10^{-3}$ mBar for a period comprised between 10-60 minutes, preferably 10-30 minute.

9. The method according to any one of claims 1 to 8, wherein said sterilization is a moist heat sterilization in autoclave, which is preferably carried out at a temperature comprised between 100°C and 130°C, in the presence of steam pressure comprised between 90 kPa and 150 kPa over atmospheric pressure for a time comprised between 10- 90 minutes, where said step e) of conditioning the leached units is carried out in water or in a buffer solution, preferably phosphate-buffered saline.

10. The method according to any one of claims 1 to 8, wherein said at least one gas-forming agent is selected from the group consisting of sodium hydrogen carbonate, potassium hydrogen carbonate, sodium glycine carbonate, ammonium hydrogen carbonate, sodium bisulfite, and sodium metabisulfite, and said aqueous medium is deionized water.

11. The method according to any one of claims 1 to 8, wherein said gas-forming agent is selected from the group consisting of calcium carbonate and sodium sulfite, and said aqueous medium is an acidic aqueous solution having a pH ranging from 0.1-3.

12. The method according to any one of claims 1 to 11, wherein said water-soluble salt is an alkali metal or an alkaline-earth metal salt, preferably selected from the group consisting of sodium chloride (NaCl), potassium chloride (KCl), potassium sulfate ($K_2SO_4$), sodium sulfate ($Na_2SO_4$), potassium dihydrogen phosphate ($KH_2PO_4$), disodium hydrogen phosphate ($Na_2HPO_4$), magnesium sulfate ($MgSO_4$), aluminum chloride ($AlCl_3$), magnesium chloride ($MgCl_2$), and potassium acetate ($KC_2H_3O_2$).

13. The method according to any one of claims 1 to 12, wherein said at least one non-water-soluble, water-swellable cross-linked poly(acrylic) polymer constitutes 20-80%, preferably 22-60%, even more preferably 30-50% by weight of the total weight of said solid mixture, and is selected from the group consisting of Carbomer, Polycarbophil and salts thereof.

14. The method according to any one of claims 1 to 13, wherein said at least one gas-forming agent constitutes 0.5%-79.5% by weight of the total weight of said solid mixture preferably 10-50%, even more preferably 20-40%, and wherein said water-soluble salt constitutes 0.5%-79.5% by weight of the total weight of said solid mixture preferably 10-50%, even more preferably 20-40%.

15. The method according to any one of claims 1 to 14, wherein said at least one first uniform solid mixture comprises a mineralizing compound selected from the group consisting of synthetic hydroxyapatite ($Ca_{10}(PO_4)_6(OH)_2$, tricalcium phosphate ($Ca_3(PO_4)_2$), hydroxyapatite ($Ca_5(PO_4)_3OH$), carbonated hydroxyapatite, wollastonite, magnesium silicate, alumina and zirconia.

**Patentansprüche**

1. Verfahren zur Herstellung eines dreidimensionalen Hydrogel-Gerüsts zur Zellkultur, das folgende Schritte aufweist:

a) Herstellen mindestens eines ersten einheitlichen festen Gemisches, aufweisend mindestens ein gasbildendes Mittel, das ausgewählt wird aus der Gruppe, die aus Natriumhydrogencarbonat, Kaliumhydrogencarbonat, Natriumglycincarbonat, Ammoniumhydrogencarbonat, Natriumhydrogensulfit, Natriumdisulfit, Calciumcarbonat und Natriumsulfit besteht, mindestens ein wasserlösliches Salz und mindestens ein nicht wasserlösliches, wasserquellfähiges vernetztes Polyacryl-Polymer;
b) Herstellen komprimierter oder kompakter Elemente ausgehend von dem mindestens einen ersten festen Gemisch;
c) Unterziehen der so erhaltenen komprimierten oder kompakten Elemente einem Erhitzen bei einer Temperatur in dem Bereich von 120°C-160°C für eine Zeit von 1-120 Minuten, bevorzugt 1-20 Minuten;
d) Auswaschen der so erhaltenen thermisch behandelten Elemente in einem wässrigen Medium;
e) Konditionieren der so erhaltenen ausgewaschenen Elemente in Wasser oder in einer wässrigen Lösung, die ausgewählt wird aus der Gruppe, die eine Pufferlösung, bevorzugt Phosphat-gepufferte Salzlösung (PBS) aufweist, und einem Zellkulturmedium;
f) Unterziehen der so erhaltenen konditionierten Elemente einer Sterilisierung.

2. Verfahren nach Anspruch 1, bei dem die in dem Schritt f) erhaltenen sterilisierten Elemente einer Lyophilisierung unterzogen werden.

3. Verfahren nach Anspruch 1 oder 2, bei dem die komprimierten oder kompakten Elemente durch direkte Kompression des festen Gemisches, in dem das mindestens eine gasbildende Mittel, das mindestens eine wasserlösliche Salz und das mindestens eine nicht-wasserlösliche, wasserquellfähige vernetzte Polyacryl-Polymer alle in Pulverform sind, hergestellt werden.

4. Verfahren nach Anspruch 1 oder 2, bei dem die komprimierten oder kompakten Elemente durch Komprimieren des festen Gemisches, bevorzugt bei einem Druck zwischen 2kPa/g und 2500 GPa/g, bevorzugter 150 kPa/g bis 25 GPa/g, für eine Zeit im Bereich von 0,1 Sekunden bis 30 Minuten, in dem das mindestens eine nicht-wasserlösliche, wasserquellfähige vernetzte Polyacryl-Polymer in Pulverform ist und das mindestens eine gasbildende Mittel und das mindestens eine wasserlösliche Salz vorher granuliert oder sphäronisiert wurden, hergestellt werden.

5. Verfahren nach einem der Ansprüche 1 bis 4, das aufweist

   - Herstellen eines zweiten einheitlichen festen Gemisches, aufweisend mindestens ein nichtwasserlösliches, wasserquellfähiges vernetztes Polyacryl-Polymer, welches dasselbe sein kann wie das erste einheitliche feste Gemisch oder davon verschieden sein kann;
   - Ausbilden einer Schicht aus dem zweiten einheitlichen festen Gemisch, Auftragen einer Schicht aus dem ersten festen Gemisch über der Schicht aus dem zweiten einheitlichen festen Gemisch und optional Auftragen einer weiteren Schicht aus dem zweiten einheitlichen festen Gemisch auf der Schicht aus dem ersten festen Gemisch;

   wobei die komprimierten oder kompakten Elemente von Schritt b) durch Komprimieren aller der Schichten hergestellt werden.

6. Verfahren nach einem der Ansprüche 1 bis 4, das aufweist

   - Herstellen eines zweiten einheitlichen festen Gemisches, aufweisend mindestens ein nichtwasserlösliches, wasserquellfähiges vernetztes Polyacryl-Polymer, das dasselbe sein kann wie das erste einheitliche feste Gemisch oder davon verschieden sein kann;
   - Herstellen eines komprimierten oder kompakten Kerns aus dem ersten einheitlichen festen Gemisch;
   - Ausbilden einer Schicht aus dem zweiten einheitlichen festen Gemisch im Inneren einer Form, die einen Boden und eine Umfangswand hat, Legen des komprimierten oder kompakten Kerns auf die Schicht, wobei der komprimierte oder kompakte Kern weniger breit ist als die Form, so dass zwischen dem Kern und der Umfangswand ein Spalt gebildet wird,
   - Füllen des Spalts mit dem zweiten einheitlichen festen Gemisch bis die obere Oberfläche des zweiten einheitlichen festen Gemisches mit der oberen Oberfläche des komprimierten oder kompakten Kerns bündig ist;

   wobei die komprimierten oder kompakten Elemente von Schritt b) durch Komprimieren des zweiten einheitlichen festen Gemisches und des komprimierten oder kompakten Kerns, die in der Form enthalten sind, hergestellt werden.

7. Verfahren nach einem der Ansprüche 1 bis 6, bei dem die in Schritt b) erhaltenen komprimierten oder kompakten Elemente teilweise beschichtet werden mit einem Film aus einem polymeren Material, das ausgewählt wird aus der Gruppe, die besteht aus Hydroxypropylcellulose (HPC), Ethylcellulose (EC), Hydroxyethylmethylcellulose (HEMC), Hydroxypropylmethylcellulose (HPMC), Methylcellulose (MC), Polyvinylalkohol (PVA), Polyvinylalkohol-acetatphthalat, Polymethacrylaten (d. h. Eudragit) und Gemischen davon.

8. Verfahren nach einem der Ansprüche 1 bis 7, bei dem nach dem Schritt d) des Auswaschens die ausgewaschenen Elemente bei einem Druck zwischen $80 \times 10^{-3}$ mBar und $120 \times 10^{-3}$ mBar für eine Dauer zwischen 10-60 Minuten, bevorzugt 10-30 Minuten, gehalten werden.

9. Verfahren nach einem der Ansprüche 1 bis 8, bei dem die Sterilisierung eine feuchte Hitzesterilisierung in einem Autoklaven ist, die bevorzugt durchgeführt wird bei einer Temperatur zwischen 100°C und 130°C, in Anwesenheit von Dampfdruck zwischen 90 kPa und 150 kPa über Atmosphärendruck für eine Zeit zwischen 10-90 Minuten, wobei der Schritt e) des Konditionierens der ausgewaschenen Elemente in Wasser oder in einer Pufferlösung, bevorzugt Phosphatgepufferter Salzlösung, durchgeführt wird.

10. Verfahren nach einem der Ansprüche 1 bis 8, bei dem das mindestens eine gasbildende Mittel ausgewählt wird aus der Gruppe, die aus Natriumhydrogencarbonat, Kaliumhydrogencarbonat, Natriumglycincarbonat, Ammoniumhydrogencarbonat, Natriumhydrogensulfit und Natriumdisulfit besteht, und das wässrige Medium deionisiertes Wasser ist.

11. Verfahren nach einem der Ansprüche 1 bis 8, bei dem das gasbildende Mittel ausgewählt wird aus der Gruppe, die aus Kalziumcarbonat und Natriumsulfit besteht, und das wässrige Medium eine saure wässrige Lösung mit einem pH im Bereich von 0,1-3 ist.

12. Verfahren nach einem der Ansprüche 1 bis 11, bei dem das wasserlösliche Salz ein Alkalimetall- oder ein Erdalkalimetall- Salz ist, das bevorzugt ausgewählt wird aus der Gruppe, die besteht aus Natriumchlorid (NaCl), Kaliumchlorid (KCl), Kaliumsulfat ($K_2SO_4$), Natriumsulfat ($Na_2SO_4$), Kaliumdihydrogenphosphat ($KH_2PO_4$), Dinatriumhydrogenphosphat ($Na_2HPO_4$), Magnesiumsulfat ($MgSO_4$), Aluminiumchlorid ($AlCl_3$), Magnesiumchlorid ($MgCl_2$) und Kaliumacetat ($KC_2H_3O_2$).

13. Verfahren nach einem der Ansprüche 1 bis 12, bei dem das mindestens eine nicht-wasserlösliche, wasserquellfähige vernetzte Polyacryl-Polymer gewichtsmäßig 20-80%, bevorzugt 22-60%, noch bevorzugter 30-50% des Gesamtgewichts des festen Gemisches ausmacht und ausgewählt wird aus der Gruppe, die aus Carbomer, Polycarbophil und Salzen davon besteht.

14. Verfahren nach einem der Ansprüche 1 bis 13, bei dem das mindestens eine gasbildende Mittel 0,5%-79,5% des Gesamtgewichts des festen Gemisches, bevorzugt 10-50%, noch bevorzugter 20-40%, ausmacht und wobei das wasserlösliche Salz 0,5%-79,5% des Gesamtgewichts des festen Gemisches, bevorzugt 10-50%, noch bevorzugter 20-40%, ausmacht.

15. Verfahren nach einem der Ansprüche 1 bis 14, bei dem das mindestens eine erste einheitliche feste Gemisch eine mineralisierende Verbindung aufweist, die ausgewählt wird aus der Gruppe, die aus synthetischem Hydroxyapatit ($Ca_{10}(PO_4)_6(OH)_2$), Tricalciumphosphat ($Ca_3(PO_4)_2$), Hydroxyapatit ($Ca_5(PO_4)_3OH$), kohlensaurem Hydroxyapatit, Wollastonit, Magnesiumsilicat, Aluminiumoxid und Zirconiumdioxid besteht.


**Revendications**

1. Procédé pour la préparation d'un échafaudage d'hydrogel tridimensionnel pour culture cellulaire, qui comprend les étapes de :

    a) préparation d'au moins un premier mélange solide uniforme comprenant au moins un agent de génération de gaz, sélectionné dans le groupe constitué par l'hydrogénocarbonate de sodium, l'hydrogénocarbonate de potassium, le carbonate de glycine de sodium, l'hydrogénocarbonate d'ammonium, le bisulfite de sodium, le métabisulfite de sodium, le carbonate de calcium et le sulfite de sodium, au moins un sel soluble dans l'eau et au moins un polymère poly(acrylique) réticulé gonflant au contact de l'eau, non soluble dans l'eau ;
    b) préparation d'unités comprimées ou compactes à partir dudit au moins un premier mélange solide ;
    c) soumission des unités comprimées ou compactes ainsi obtenues à un chauffage à une température comprise entre 120°C-160°C, pendant une durée de 1-120 minutes, de préférence de 1-20 minutes ;
    d) lixiviation des unités traitées thermiquement ainsi obtenues dans un milieu aqueux ;
    e) conditionnement des unités lixiviées ainsi obtenues dans de l'eau ou dans une solution aqueuse sélectionnée dans le groupe comprenant une solution tampon, de préférence une solution saline tamponnée au phosphate (PBS) et un milieu de culture cellulaire ;
    f) soumission des unités conditionnées ainsi obtenues à une stérilisation.

2. Procédé selon la revendication 1, dans lequel les unités stérilisées ainsi obtenues dans ladite étape f) sont soumises à une lyophilisation.

3. Procédé selon la revendication 1 ou 2, dans lequel lesdites unités comprimées ou compactes sont préparées par compression directe dudit mélange solide, dans lequel ledit au moins un agent de génération de gaz, ledit au moins un sel soluble dans l'eau ledit au moins un polymère poly(acrylique) réticulé gonflant au contact de l'eau, non soluble dans l'eau sont tous sous forme de poudre.

4. Procédé selon la revendication 1 ou 2, dans lequel lesdites unités comprimées ou compactes sont préparées par compression dudit mélange solide, de préférence à une pression comprise entre 2 kPa/g et 2500 GPa/g, de préférence encore entre 150 kPa/g à 25 GPa/g, pendant une période allant de 0,1 seconde à 30 minutes, dans lequel ledit au moins un polymère poly(acrylique) réticulé gonflable au contact de l'eau, non soluble dans l'eau est sous forme de poudre et ledit au moins un agent de génération de gaz et ledit au moins un sel soluble dans l'eau ont précédemment été granulés ou sphéronisés.

5. Procédé selon l'une quelconque des revendications 1 à 4, qui comprend

- la préparation d'un second mélange solide uniforme comprenant au moins un polymère poly(acrylique) réticulé gonflable au contact de l'eau, non soluble dans l'eau, qui peut être identique audit premier mélange solide uniforme ou différent de celui-ci ;
- la formation d'une couche dudit second mélange solide uniforme, le dépôt d'une couche dudit premier mélange solide sur ladite couche du second mélange solide uniforme et éventuellement le dépôt d'une couche supplémentaire dudit second mélange solide uniforme sur ladite couche du premier mélange solide ;

dans lequel lesdites unités comprimées ou compactes de l'étape b) sont préparées en comprimant toutes lesdites couches.

6. Procédé selon l'une quelconque des revendications 1 à 4, qui comprend

- la préparation d'un second mélange solide uniforme comprenant au moins un polymère poly(acrylique) réticulé, gonflable au contact de l'eau, non soluble dans l'eau, qui peut être identique audit premier mélange solide uniforme ou différent de celui-ci ;
- la préparation d'un noyau comprimé ou compact à partir dudit premier mélange solide uniforme ;
- la formation d'une couche dudit second mélange solide uniforme à l'intérieur d'un moule, ayant un fond et une paroi périphérique, le dépôt dudit noyau comprimé ou compact sur ladite couche, le noyau comprimé ou compact étant moins large que le moule, de sorte qu'un espace est formé entre le noyau et la paroi périphérique,
- le remplissage dudit espace avec ledit second mélange solide uniforme, jusqu'à ce que la surface supérieure dudit second mélange solide uniforme affleure la surface supérieure dudit noyau comprimé ou compact ;

dans lequel lesdites unités comprimées ou compactes de l'étape b) sont préparées en comprimant ledit second mélange solide uniforme et ledit noyau comprimé ou compact contenu dans ledit moule.

7. Procédé selon l'une quelconque des revendications 1 à 6, dans lequel les unités comprimées ou compactes obtenues à l'étape b) sont partiellement revêtues d'un film réalisé en un matériau polymérique sélectionné dans le groupe constitué par l'hydroxypropylcellulose (HPC), l'éthylcellulose (EC), l'hydroxyéthylméthylcellulose (HEMC), l'hydroxypropylméthylcellulose (HPMC), la méthylcellulose (MC), l'alcool polyvinylique (PVA), l'acétate phtalate d'alcool polyvinylique, les polyméthacrylates (c'est-à-dire Eudragit) et des mélanges de ceux-ci.

8. Procédé selon l'une quelconque des revendications 1 à 7, dans lequel après ladite étape d) de lixiviation, les unités lixiviées sont maintenues à une pression comprise entre $80 \times 10^{-3}$ mBar et $120 \times 10^{-3}$ mBar pendant une période comprise entre 10-60 minutes, de préférence 10-30 minutes.

9. Procédé selon l'une quelconque des revendications 1 à 8, dans lequel ladite stérilisation est une stérilisation par chaleur humide en autoclave, qui est de préférence effectuée à une température comprise entre 100°C et 130°C, en présence d'une pression de vapeur comprise entre 90 kPa et 150 kPa au-dessus de la pression atmosphérique pendant une durée comprise entre 10 et 90 minutes, où ladite étape e) de conditionnement des unités lixiviées est effectuée dans de l'eau ou dans une solution tampon, de préférence saline et tamponnée au phosphate.

10. Procédé selon l'une quelconque des revendications 1 à 8, dans lequel ledit au moins un agent de génération de gaz est sélectionné dans le groupe constitué de l'hydrogénocarbonate de sodium, l'hydrogénocarbonate de potassium, le carbonate de glycine de sodium, l'hydrogénocarbonate d'ammonium, le bisulfite de sodium, et le métabisulfite de sodium, et ledit milieu aqueux est de l'eau désionisée.

11. Procédé selon l'une quelconque des revendications 1 à 8, dans lequel ledit agent de génération de gaz est sélectionné dans le groupe constitué de carbonate de calcium et de sulfite de sodium, et ledit milieu aqueux est une solution aqueuse acide ayant un pH compris entre 0,1 et 3.

12. Procédé selon l'une quelconque des revendications 1 à 11, dans lequel ledit sel soluble dans l'eau est un sel de métal alcalin ou de métal alcalino-terreux, de préférence sélectionné dans le groupe constitué par le chlorure de sodium (NaCl), le chlorure de potassium (KCl), le sulfate de potassium ($K_2SO_4$), le sulfate de sodium ($Na_2SO_4$), le dihydrogénophosphate de potassium ($KH_2PO_4$), l'hydrogénophosphate disodique ($Na_2HPO_4$), le sulfate de magnésium ($MgSO_4$), le chlorure d'aluminium ($AlCl_3$), le chlorure de magnésium ($MgCl_2$) et l'acétate de potassium ($KC_2H_3O_2$).

13. Procédé selon l'une quelconque des revendications 1 à 12, dans lequel ledit au moins un polymère poly(acrylique) réticulé gonflable au contact l'eau, non soluble dans l'eau, constitue 20-80 %, de préférence 22-66 %, encore plus préférablement 30-50 % en poids du poids total dudit mélange solide ; et est sélectionné dans le groupe constitué par le Carbomer, le Polycarbophile et des sels de ceux-ci.

14. Procédé selon l'une quelconque des revendications 1 à 13, dans lequel ledit au moins un agent de génération de gaz constitue 0,5 %-79,5 % en poids du poids total dudit mélange solide, de préférence 10-50 %, encore plus préférablement 20-40 %, et dans lequel ledit sel soluble dans l'eau constitue 0,5-79,5 % en poids du poids total dudit mélange solide, de préférence 10-50 %, encore plus préférablement 20-40 %.

15. Procédé selon l'une quelconque des revendications 1 à 14, dans lequel ledit au moins un premier mélange solide uniforme comprend un composé minéralisant sélectionné dans le groupe constitué par l'hydroxyapatite synthétique ($Ca_{10}(PO_4)_6(OH)_2$), le phosphate tricalcique ($Ca_3(PO_4)_2$), l'hydroxyapatite ($Ca_5(PO_4)_3OH$), l'hydroxyapatite carbonatée, la wollastonite, le silicate de magnésium, l'alumine et la zircone.

FIGURE 1A

FIGURE 1B

FIGURE 2

**FIGURE 3**

**FIGURE 4**

**FIGURE 5A**

**FIGURE 5B**

**FIGURE 6A**

**FIGURE 6B**

**FIGURE 6C**

**FIGURE 7A**

**FIGURE 7B**

FIGURE 8A

FIGURE 8B

FIGURE 9A

FIGURE 9B

FIGURE 9C

**FIGURE 10A**

**FIGURE 10B**

**FIGURE 11**

**FIGURE 12**

**FIGURE 13**

**FIGURE 14**

**FIGURE 15**

**FIGURE 16**

**FIGURE 17A**

**FIGURE 17B**

**FIGURE 18**

**FIGURE 19**

**FIGURE 20**

**FIGURE 21.**

**FIGURE 22**

**FIGURE 23**

**FIGURE 24**

**FIGURE 25**

**FIGURE 26**

**FIGURE 27**

**FIGURE 28**

**FIGURE 29 A**

**FIGURE 29 B**

**FIGURE 30 A**

**FIGURE 30 B**

**FIGURE 31A**

**FIGURE 31B**

**FIGURE 32 A**

**FIGURE 32 B**

**FIGURE 33A**

**FIGURE 33B**

**FIGURE 34**

**FIGURE 35A**

**FIGURE 35B**

**FIGURE 36**

**FIGURE 37**

**FIGURE 38**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2016168196 A1
- US 20070098799 A
- US 5902741 A
- WO 2005037144 A2, Liu, G. M.
- WO 2007103135 A2, Sheetz, M. P.; Hone, J. C.; Tanase, M.; Wind, S. J
- US 20080194010 A1, Liu, Q
- KR 2012111380 A, Shin, J. U.; Shin, J. W.; Park, S. H
- WO 2013172788 A1
- US 8283028 B2, Yue, Z.; Wen, F.; Yu, H

### Non-patent literature cited in the description

- **NASIM ANNABI et al.** Controlling the porosity and Microarchitecture of Hydrogels for Tissue Engineering. *Tissue Engineering part B-Reviews,* August 2010, vol. 16 (4), 371-383
- **SHENGTON SUN et al.** Hydrogels from amorphous calcium carbonate and polyacrylic acid: bio-inspired materials for ''mineral plastics. *Angewandte Chemie International Edition,* 22 July 2016, vol. 55 (39), 11765-11769
- **JUNMIN ZHU.** Bioactive modification of poly(ethylene glycol) hydrogels for tissue engineering. *Biomaterials,* June 2010, vol. 31 (17), 4639-4656
- **FANG REN.** *Synthesis of gold nanoparticle-hydrogel nanocomposites with controlled cytotoxicity and unique cell-adhesive properties,* 09 September 2016, 1-132, https://depositonce.tu-berlin.de/bitstream/11303/5988/4/ren_fang.pdf
- Handbook of Pharmaceutical Excipients. 2009
- *CHEMICAL ABSTRACTS,* 9003-01-4
- *CHEMICAL ABSTRACTS,* 9007-20-9
- *CHEMICAL ABSTRACTS,* 9003-97-8
- **LEE J ; CUDDIHY MJ ; KOTOV NA.** Three-dimensional cell culture matrices: State of the art. *Tissue Engineering Part B-Reviews,* 2008, vol. 14 (1), 61-86
- **ANDREI G.** Three-dimensional culture models for human viral diseases and antiviral drug development. *Antiviral Research,* 2006, vol. 71 (2-3), 96-107
- **RAVI M ; PARAMESH V ; KAVIYA SR ; ANURADHA E ; SOLOMON FDP.** 3D Cell Culture Systems: Advantages and Applications. *Journal Of Cellular Physiology,* 2015, vol. 230 (1), 16-26
- **XU X ; FARACH-CARSON MC ; JIA X.** Three-dimensional in vitro tumor models for cancer research and drug evaluation. *Biotechnology Advances,* 2014, vol. 32 (7), 1256-1268
- **SMITH SJ ; WILSON M ; WARD JH ; RAHMAN CV ; PEET AC ; MACARTHUR DC ; ROSE FRAJ ; GRUNDY RG ; RAHMAN R.** Recapitulation of Tumor Heterogeneity and Molecular Signatures in a 3D Brain Cancer Model with Decreased Sensitivity to Histone Deacetylase Inhibition. *Plos One,* 2012, vol. 7, 12
- **PICOLLET-D'HAHAN N ; DOLEGA ME ; LIGUORI L ; MARQUETTE C ; LE GAC S ; GIDRO X ; MARTIN DK.** A 3D Toolbox to Enhance Physiological Relevance of Human Tissue Models. *Trends in Biotechnology,* 2016, vol. 34 (9), 757-769
- **CHUAH JKC ; ZINK D.** Stem cell-derived kidney cells and organoids: Recent breakthroughs and emerging applications. *Biotechnology Advances,* 2017, vol. 35 (2), 150-167
- **PRESTWICH GD.** Evaluating drug efficacy and toxicology in three dimensions: Using synthetic extracellular matrices in drug discovery. *Accounts of Chemical Research,* 2008, vol. 41 (1), 139-148
- **YANG J ; SHI GX ; BEI JZ ; WANG SG ; CAO YL ; SHANG QX ; YANG GG ; WANG WJ.** Fabrication and surface modification of macroporous poly(L-lactic acid) and poly(L-lactic-co-glycolic acid) (70/30) cell scaffolds for human skin fibroblast cell culture. *Journal of Biomedical Materials Research,* 2002, vol. 62 (3), 438-446
- **KLEIN F ; RICHTER B ; STRIEBEL T ; FRANZ CM ; VON FREYMANN G ; WEGENER M ; BASTMEYER M.** Two-Component Polymer Scaffolds for Controlled Three-Dimensional Cell Culture. *Advanced Materials,* 2011, vol. 23 (11), 1341-1345

- **NIE L ; CHEN D ; SUO JP ; ZOU P ; FENG SB ; YANG Q ; YANG SH ; YE SN.** Physicochemical characterization and biocompatibility in vitro of biphasic calcium phosphate/polyvinyl alcohol scaffolds prepared by freeze-drying method for bone tissue engineering applications. *Colloids and Surfaces B-Biointerfaces,* 2012, vol. 100, 169-176
- **BOKHARI M ; CARNACHAN RJ ; PRZYBORSKI SA ; CAMERON NR.** Emulsion-templated porous polymers as scaffolds for three dimensional cell culture: effect of synthesis parameters on scaffold formation and homogeneity. *J Mater Chem,* 2007, vol. 17 (38), 4088-4094
- **RUEDINGER F ; LAVRENTIEVA A ; BLUME C ; PEPELANOVA I ; SCHEPER T.** Hydrogels for 3D mammalian cell culture: a starting guide for laboratory practice. *Applied Microbiology and Biotechnology,* 2015, vol. 99 (2), 623-636
- **ROUQUEROL J ; AVNIR D ; FAIRBRIDGE CW ; EVERETT DH ; HAYNES JH ; PERNICONE N ; RAMSAY JDF ; SING KSW ; UNGER KK.** Recommendations For The Characterization Of Porous Solids. *Pure and Applied Chemistry,* 1994, vol. 66 (8), 1739-1758
- **CALIARI SR ; BURDICK JA.** A practical guide to hydrogels for cell culture. *Nat Methods,* 2016, vol. 13 (5), 405-414
- **ZHANG D ; PEKKANEN-MATTILA M ; SHAHSAVANI MO ; FALK A ; TEIXEIRA AI ; HERLAND A.** A 3D Alzheimer's disease culture model and the induction of P21-activated kinase mediated sensing in iPSC derived neurons. *Biomaterials,* 2014, vol. 35 (5), 1420-1428
- **WU X ; LIU Y ; LI X ; WEN P ; ZHANG Y ; LONG Y ; WANG X ; GUO Y ; XING F ; GAO J.** Preparation of aligned porous gelatin scaffolds by unidirectional freeze-drying method. *Acta Biomaterialia,* 2010, vol. 6 (3), 1167-1177
- **ANNABI N ; NICHOL JW ; ZHONG X ; JI CD ; KOSHY S ; KHADEMHOSSEINI A ; DEHGHANI F.** Controlling the Porosity and Microarchitecture of Hydrogels for Tissue Engineering. *Tissue Engineering Part B-Reviews,* 2010, vol. 16 (4), 371-383
- **NAM YS ; YOON JJ ; PARK TG.** A novel fabrication method of macroporous biodegradable polymer scaffolds using gas foaming salt as a porogen additive. *Journal of Biomedical Materials Research,* 2000, vol. 53 (1), 1-7
- **NIETO-SUAREZ M ; LOPEZ-QUINTELA MA ; LAZZARI M.** Preparation and characterization of cross-linked chitosan/gelatin scaffolds by ice segregation induced self-assembly. *Carbohydr Polym,* 2016, vol. 141, 175-183
- **BOTCHWEY EA ; POLLACK SR ; LEVINE EM ; LAURENCIN CT.** Bone tissue engineering in a rotating bioreactor using a microcarrier matrix system. *Journal of Biomedical Materials Research,* 2001, vol. 55 (2), 242-253
- **TENG WQ ; LONG TJ ; ZHANG QR ; YAO K ; SHEN TT ; RATNER BD.** A tough, precision-porous hydrogel scaffold: Ophthalmologic applications. *Biomaterials,* 2014, vol. 35 (32), 8916-8926
- **OSTROWSKA-CZUBENKO J ; GIERSZEWSKA-DRUZYNSKA M.** Effect of ionic crosslinking on the water state in hydrogel chitosan membranes. *Carbohydr Polym,* 2009, vol. 77 (3), 590-598
- **HOPKINS AM ; DE LAPORTE L ; TORTELLI F ; SPEDDEN E ; STAII C ; ATHERTON TJ ; HUBBELL JA ; KAPLAN DL.** Silk Hydrogels as Soft Substrates for Neural Tissue Engineering. *Adv FunctMater,* 2013, vol. 23 (41), 5140-5149
- **HAYWARD AS ; SANO N ; PRZYBORSKI SA ; CAMERON NR.** Acrylic-Acid-Functionalized Poly-HIPE Scaffolds for Use in 3D Cell Culture. *Macromol Rapid Commun,* 2013, vol. 34 (23-24), 1844-1849
- **SON JS ; PARK K ; OH S ; KIM J-J ; HAN DK.** Preparation and characterization of porous heparin-immobilized PLGA scaffolds using novel solid hydrogen peroxide for cartilage regeneration. *Tissue Eng Regen Med,* 2008, vol. 5 (3), 528-534
- **ZHANG J ; YANG Z ; LI C ; DOU Y ; LI Y ; THOTE T ; WANG D-A ; GE Z.** Cells Behave Distinctly Within Sponges and Hydrogels Due to Differences of Internal Structure. *Tissue Engineering Part A,* 2013, vol. 19 (19-20), 2166-2175
- Multifunction Co-culture Model for Evaluating Cell-Cell Interactions. **BOGDANOWICZ DR ; LU HH.** Biomimetics and Stem Cells: Methods and Protocols. Springer, 29-36
- **D.P. MOHANTY ; S. MOHAPATRA ; S. MISRA ; P.S. SAHUB.** Milk derived bioactive peptides and their impact on human health - A review. *Saudi J Biol Sci.,* September 2016, vol. 23 (5), 577-583
- **KRUGER, T. E. ; MILLER, A. H. ; WANG, J. X.** Collagen Scaffolds in Bone Sialoprotein-Mediated Bone Regeneration. *Sci. World J,* 2013
- **FERRAZ, M.P. ; F.J. MONTEIRO ; C.M. MANUEL.** Hydroxyapatite nanoparticles: A review of preparation methodologies. *J Appl Biomater Biomech,* 2004, vol. 2 (2), 74-80
- **CUNNIFFE, G.M. et al.** The synthesis and characterization of nanophase hydroxyapatite using a novel dispersant-aided precipitation method. *Journal of Biomedical Materials Research Part A,* 2010, vol. 95A (4), 1142-1149
- **WANG, P.P. et al.** Effects of synthesis conditions on the morphology of hydroxyapatite nanoparticles produced by wet chemical process. *Powder Technology,* 2010, vol. 203 (2), 315-321
- **BOUYER, E. ; F. GITZHOFER ; M.I. BOULOS.** Morphological study of hydroxyapatite nanocrystal Suspension. *Journal of Materials Science-Materials in Medicine,* 2000, vol. 11 (8), 523-531

- **KOTHAPALLI, C. et al.** Influence of temperature and concentration on the sintering behavior and mechanical properties of hydroxyapatite. *Acta Materialia,* 2004, vol. 52 (19), 5655-5663
- **TAMPIERI, A. ; SPRIO, S. ; SANDRI, M. ; VALENTINI, F.** Mimicking natural bio-mineralization processes: A new tool for osteochondral scaffold development. *Trends in Biotechnology,* 2011, vol. 29 (10), 526-535
- **KRETLOW, J. D. ; MIKOS, A. G.** Review: Mineralization of synthetic polymer scaffolds for bone tissue engineering. *Tissue Engineering,* 2007, vol. 13 (5), 927-938
- **NIU, J.L.** Hydrothermal synthesis of nano-crystalline hydroxyapatite. *Bioceramics,* 2007, vol. 19, 247-250
- **YOSHIMURA, M ; K. BYRAPPA.** Hydrothermal processing of materials: past, present and future. *Journal of Materials Science,* 2008, vol. 43 (7), 2085-2103
- **MANAFI, S.A. et al.** Synthesis of nano-hydroxyapatite under a sonochemical/hydrothermal condition. *Biomedical Materials,* 2008, vol. 3 (2
- **CHAUDHRY, A.A. et al.** Instant nano-hydroxyapatite: a continuous and rapid hydrothermal synthesis. *Chemical Communications,* 2006, vol. 21, 2286-2288
- **MANAFI, S.A. et al.** Synthesis of nano-hydroxyapatite under a sonochemical/hydrothermal condition. *Biomedical Materials,* 2008, vol. 3, 2
- **GUO, X.Y ; P. XIAO.** Effects of solvents on properties of nanocrystalline hydroxyapatite produced from hydrothermal process. *Journal of the European Ceramic Society,* 2006, vol. 26 (15), 3383-3391
- **WANG, Y.J. et al.** Investigations on the formation mechanism of hydroxyapatite synthesized by the solvothermal method. *Nanotechnology,* 2006, vol. 17 (17), 4405-4412
- **ZHANG, S.Y. et al.** Kinetic studies on the synthesis of hydroxyapatite nanowires by solvothermal methods. *Australian Journal of Chemistry,* 2007, vol. 60 (2), 99-104
- **MA, M.G ; J.F. ZHU.** Solvothermal Synthesis and Characterization of Hierarchically Nanostructured Hydroxyapatite Hollow Spheres. *European Journal of Inorganic Chemistry,* 2009, (36), 5522-5526
- **YANG, C. et al.** Solvothermal synthesis and characterization of Ln (Eu(3+),Tb(3+)) doped hydroxyapatite. *Journal of Colloid and Interface Science,* 2008, vol. 328 (1), 203-210
- **LIU, D.M. ; T. TROCZYNSKI ; W.J. TSENG.** Water-based sol-gel synthesis of hydroxyapatite: process development. *Biomaterials,* 2001, vol. 22 (13), 1721-1730
- **BEZZI, G. et al.** A novel sol-gel technique for hydroxyapatite preparation. *Materials Chemistry and Physics,* 2003, vol. 78 (3), 816-824
- **WENG, W.J. et al.** The effect of citric acid addition on the formation of sol-gel derived hydroxyapatite. *Materials Chemistry and Physics,* 2002, vol. 74 (1), 92-97
- **AGRAWAL, K. et al.** Synthesis of HA by various sol-gel techniques and their comparison: a review. *National Conference on Advancements and Futuristic Trends in Mechanical and Materials Engineering,* 2011
- **ZHANG, H ; COOPER, A. I.** Synthesis and applications of emulsion-templated porous materials. *Soft Matter,* 2005, vol. 1, 107-113
- **LIM, G. K. et al.** Processing of hydroxyapatite via microemulsion and emulsion routes. *Biometerials,* 1997, vol. 18, 1433-1439
- **YANG, J. H. et al.** Synthesis of spherical hydroxyapatite granules with interconnected pore channels using camphene emulsion. *J Biomedical Materials Research Part B,* 2011, vol. 99B (1), 150-157
- **YANG et al.** *Biomineralization of Natural Collagenous Nanofibrous Membranes and Their Potential Use in Bone Tissue Engineering J Biomed Nanotechnol,* 2015, vol. 11 (3), 447-456
- **PAWELEC et al.** Biomineralization of Recombinant Peptide Scaffolds: Interplay among Chemistry, Architecture, and Mechanics ACS Biomater. *Sci. Eng.,* 2017, vol. 3 (6), 1100-1108
- **XU et al.** Biomimetic mineralization. *J. Mater. Chem.,* 2007, vol. 17, 415-449